# FASCICULE DE BREVET EUROPEEN

(11) **EP 3 356 441 B1**
(45) Date de publication et mention de la délivrance du brevet: **22.05.2024**
(21) Numéro de dépôt: 16774693.2
(22) Date de dépôt: 30.09.2016
(51) Int. Cl.: C08G 18/72, C08G 18/78, C08G 18/79, C08G 18/09, C08G 18/36, C08G 18/28, C07C 275/60, C07C 275/62, C08G 18/62, C08G 18/40, C08G 18/42

(54) **COMPOSITION POLYISOCYANATE BIURET**
BIURET-POLYISOCYANAT-ZUSAMMENSETZUNG
BIURET POLYISOCYANATE COMPOSITION

(30) Priorité: 30.09.2015 FR 1559271
(43) Date de publication de la demande: 08.08.2018
(73) Titulaire: Vencorex France, 69800 Saint-Priest (FR)
(72) Inventeur: BERNARD, Jean-Marie, 69440 Saint-Laurent d'Agny (FR); OLIER, Philippe, 69007 Lyon (FR)
(74) Mandataire: Dennemeyer & Associates S.A.
(86) Numéro de dépôt international: PCT/EP2016/073426
(87) Numéro de publication internationale: WO 2017/055552

(56) Documents cités:
- FR-A1- 2 372 853
- FR-A1- 2 777 894
- US-A1- 2005 107 565

## Description

L'invention concerne le domaine des compositions de polyisocyanates pour la préparation de revêtements et d'adhésifs. La composition selon l'invention combine un composé polyisocyanate à motifs biuret, un composé diluant réactif polaire, protique, un de leurs composés d'addition et un composé diluant réactif apolaire, aprotique. Les compositions de polyisocyanates selon l'invention peuvent être utilisées à de hauts degrés d'extrait sec. L'invention concerne également un procédé de préparation de la composition selon l'invention ainsi que son utilisation pour la préparation d'un revêtement ou d'un adhésif.

De manière générale dans le domaine des compositions de polyisocyanates pour la préparation de revêtements et d'adhésifs, les propriétés recherchées par rapport aux compositions connues sont la réduction de la viscosité ainsi que l'augmentation de la fonctionnalité. On recherche également la stabilité de la composition ainsi que la compatibilité avec les autres substances chimiques utilisées lors de la préparation des revêtements et des adhésifs. Par exemple la demande FR2372853 A1 a pour objet la préparation de polyisocyanate-biurets à faible viscosité, peu colorés et stables.

On connait de nombreuses compositions de polyisocyanates pour la préparation de revêtements ou pour la préparation d'adhésifs. Toutefois, ces compositions présentent des inconvénients liés à leur viscosité souvent trop élevée. La viscosité trop élevée des compositions de polyisocyanates de l'état de la technique peut empêcher leur utilisation comme durcisseur pour des applications polyuréthane ou polyurée. Une viscosité trop élevée peut également empêcher leur utilisation pour des applications à des pourcentages d'extrait sec élevés ou pour des applications à 100 % d'extrait sec.

Afin de réduire la viscosité des compositions de l'état de la technique, on utilise régulièrement des solvants. L'utilisation de solvants conduit à des problèmes de coûts mais également à des problèmes environnementaux ou à des problèmes de toxicité.

Les compositions de l'état de la technique possèdent des niveaux de fonctionnalité parfois insuffisants, notamment pour les compositions de faible viscosité. Les compositions de polyisocyanates de l'état de la technique présentent également des problèmes lors de leur mise en oeuvre. Un problème rencontré concerne le temps de démoussage élevé lors de la mise en oeuvre de telles compositions.

La compatibilité des compositions de polyisocyanates de l'état de la technique, notamment avec des polyols polaires, par exemple avec des polyesters, est également problématique.

Le temps de séchage des compositions de revêtement préparées au moyen de compositions de polyisocyanates de l'état de la technique est souvent trop long et donc problématique.

Les procédés de préparation des compositions de l'état de la technique posent également des problèmes, en particulier des problèmes liés à l'utilisation de différents catalyseurs lors de synthèses successives.

Il est donc important de pouvoir disposer de procédés de préparation qui sont simples à mettre en oeuvre et qui soient plus économiques que les procédés de l'état de la technique. Il est également important de disposer de procédés qui permettent des rendements élevés. Ainsi, il existe un besoin de disposer de compositions de polyisocyanates pour la préparation de revêtements ou pour la préparation d'adhésifs qui permettent d'apporter des solutions aux problèmes des compositions de l'état de la technique. Des besoins analogues existent pour des procédés de préparation de compositions de polyisocyanates pour la préparation de revêtement ou pour la préparation d'adhésifs.

L'invention fournit une composition qui permet d'apporter des solutions à tout ou partie des problèmes des compositions de polyisocyanates pour la préparation de revêtement ou pour la préparation d'adhésifs de l'état de la technique.

L'invention concerne donc une composition dont la fonctionnalité isocyanate moyenne est supérieure à 2,5, et dont le titre NCO est compris entre 10 et 25% en poids, comprenant
- au moins un composé polyisocyanate à motifs biuret (a) ;
- au moins un composé diluant réactif polaire, protique (b), choisi parmi les composés polyisocyanates à motifs allophanate, et dont la viscosité mesurée à 25 °C est inférieure à 500 mPa.s ;
- au moins un composé d'addition (c) d'un composé (a) et d'un composé (b) et
- au moins un composé diluant réactif aprotique (d) choisi parmi un composé de formule (IV), un composé de formule (V), un composé de formule (VI) et un composé de formule (VII) tels que définis ci-après.

De préférence, le composé (d) est un composé apolaire aprotique.

Le composé polyisocyanate à motifs biuret (a) selon l'invention comprend donc des fonctions biuret. Ces fonctions biurets peuvent notamment être présentes au sein de composés biurets issus de 3 monomères (n=3), de composés bis-biurets issus de 5 monomères (n=5), de composés biurets issus de plus de 5 monomères (n>5) ou de composés biurets dimères,

Par composé diluant réactif polaire protique (b), on entend un composé comprenant au moins une et de préférence deux fonctions isocyanates et une fonction caractérisée par l'enchaînement -N-H-C(=O)-N(-)-C(=O)- capable d'établir une liaison hydrogène entre l'hydrogène porté par l'azote et l'oxygène du groupe carbonyle -(C=O)- non-vicinal de l'atome d'azote porteur de l'atome d'hydrogène.

Le composé d'addition (c) selon l'invention comprend au moins une fonction biuret et au moins une fonction caractéristique du diluant réactif polaire protique (b) ainsi qu'au moins 4 unités monomères issues du ou des composés isocyanates de départ, de préférence des composés diisocyanates. Le composé (c) combine donc au moins une fonction biuret et au moins une des fonctions choisies parmi les fonctions allophanates. La fonctionnalité NCO du composé d'addition (c) est au moins égale à 2, de préférence supérieure à 2.

Le composé d'addition (c) selon l'invention comprend donc des fonctions biuret et au moins des fonctions allophanates.

La viscosité de la composition selon l'invention peut varier assez largement. Avantageusement, la viscosité mesurée à 25 °C, de la composition selon l'invention est inférieure à 30 000 mPa.s, de préférence inférieure à 10 000 mPa.s., de préférence inférieure à 5 000 mPa.s, de préférence inférieure à 2 000 mPa.s, de manière avantageuse inférieure à 1 500 mPa.s, avantageusement inférieure à 1 200 mPa.s, de manière encore plus préférée 1 000 mPa.s.

De manière également préférée, la composition selon l'invention possède une fonctionnalité isocyanate moyenne supérieure à 2,75. Plus préférentiellement, cette fonctionnalité isocyanate moyenne est supérieure à 2,8, de préférence supérieure à 3.

La composition selon l'invention comprend des fonctions isocyanates (NCO) issues des différents composés présents et possède donc un titre NCO pouvant varier assez largement. Le titre NCO est compris entre 10 et 25 % en poids, de préférence entre 15 et 24 % en poids.

Selon l'invention, le composé (b) possède une fonctionnalité NCO égale à 2 +/-0,5, de préférence égale à 2 +/- 0,3, plus préférentiellement égale à 2 +/- 0,2. Selon l'invention, la fonctionnalité NCO moyenne du composé (b) peut être choisie parmi une fonctionnalité NCO allant de 1,9 à 2,5 ; une fonctionnalité NCO allant de 1,9 à 2,3 ; une fonctionnalité NCO allant 1,9 à 2,2 ; une fonctionnalité NCO allant de 1,9 à 2,1 ; une fonctionnalité NCO allant de 2 à 2,5 ; une fonctionnalité NCO allant de 2 à 2,3 ; une fonctionnalité NCO allant 2 à 2,2.

Les quantités de composés (a), (b), (c) et (d) peuvent varier. De manière préférée, la composition selon l'invention comprend de 40 à 90 % en poids, de préférence de 40 à 80 % en poids, de préférence de 40 à 70 % en poids, avantageusement de 40 à 60 % en poids et encore plus avantageusement de 40 à 50 % en poids de composé (a).

De manière préférée, la composition selon l'invention comprend de 2 à 50% en poids, de préférence de 5 à 50% en poids de composé (b).

De manière préférée, la composition selon l'invention comprend de 40 à 90 % en poids, de préférence de 40 à 80 % en poids, de préférence de 40 à 70 % en poids, avantageusement de 40 à 60 % en poids et encore plus avantageusement de 40 à 50 % en poids de composé (a) et de 2 à 50% en poids, de préférence de 5 à 50% en poids de composé (b).

De manière préférée, la composition selon l'invention comprend de 0,5 à 40% en poids, de préférence de 0,5 à 20% en poids, de préférence de 0,5 à 10% en poids de composé (c). De manière préférée, la composition selon l'invention comprend de 1 à 20% en poids de composé (d).

De manière avantageuse, la composition selon l'invention comprend :
∘ de 40 à 90 % en poids, de préférence de 40 à 80 % en poids, de préférence de 40 à 70 % en poids, avantageusement de 40 à 60 % en poids et encore plus avantageusement de 40 à 50 % en poids de composé (a) ; et/ou
∘ de 2 à 50 % en poids ou de 5 à 50 % en poids de composé (b) ; et/ou
∘ de 0,5 à 40% en poids, de préférence de 0,5 à 20 % en poids ou de 0,5 à 10 % en poids de composé (c) et/ou
∘ de 1 à 20 % en poids de composé (d).

Le composé (a) de la composition selon l'invention est avantageusement préparé à partir d'un composé monomère isocyanate choisi parmi 2-methyl-pentane-diisocyanate (MPDI), hexamethylène-diisocyanate (HDI), tétraméthylène-diisocyanate, pentaméthylène-diisocyanate, octaméthylène-diisocyanate, butylène-diisocyanate, octylène-diisocyanate, triméthtylhexane diisocyanate, dodécane-diisocyanate, undécane-diisocyanate, 2,2,4-tris-methyl-hexamethylene-diisocyanate, 2,4,4-tris-méthyl-hexaméthylène-diisocyanate, 1,8-diisocyanato-4-isocyanato-méthyl-octane, 1 -decane-triisocyanate, isophorone-diisocyanate (IPDI), xylylènediphenyl-diisocyanate (XDI), meta-xylylènediphenyl-diisocyanate (MXDI), para-xylylènediphenyl-diisocyanate (PXDI), méthylène-dicyclohexyl-4-4'-diisocyanate (H₁₂MDI), hexahydrogeno-tolyl-diisocyanate (H₆TDI), dérivés de lysine diisocyanate, les BIC, les NBDI. De manière préférée, le monomère utilisé pour préparer composé polyisocyanate à motifs biuret (a) de la composition selon l'invention est choisi parmi les composés monomères diisocyanates aliphatiques linéaires tels que 2-methyl-pentane-diisocyanate (MPDI), hexamethylene-diisocyanate (HDI), tetramethylene-diisocyanate, pentamethylene-diisocyanate, octamethylene-diisocyanate, butylene-diisocyanate, octylene-diisocyanate, trimethtylhexane diisocyanate, dodécane-diisocyanate, undécane-diisocyanate, 2,2,4-tris-methyl-hexamethylene-diisocyanate, 2,4,4-tris-methyl-hexamethylene-diisocyanate, 1,8-diisocyanato-4-isocyanato-methyl-octane, 1-decane-triisocyanate. Le monomère isocyanate préféré est l'hexamethylene-diisocyanate (HDI).

Le composé (b) de la composition selon l'invention est avantageusement préparé à partir d'un composé monomère isocyanate choisi parmi 2-methyl-pentane-diisocyanate (MPDI), hexaméthylène-diisocyanate (HDI), tétramethylène-diisocyanate, pentamethylène-diisocyanate, octaméthylène-diisocyanate, butylène-diisocyanate, octylène-diisocyanate, triméthtylhexane diisocyanate, dodécane-diisocyanate, undécane-diisocyanate, 2,2,4-tris-méthyl-hexaméthylène-diisocyanate, 2,4,4-tris-méthyl-hexaméthylène-diisocyanate, 1,8-diisocyanato-4-isocyanato-méthyl-octane, 1 -décane-triisocyanate, isophorone-diisocyanate (IPDI), xylylènediphenyl-diisocyanate (XDI), méta-xylylènediphenyl-diisocyanate (MXDI), para-xylylènediphenyl-diisocyanate (PXDI), méthylène-dicyclohexyl-4-4'-diisocyanate (H₁₂MDI), hexahydrogéno-tolyl-diisocyanate (H₆TDI), dérivés de lysine diisocyanate, les BIC, les NBDI. De manière préférée, le monomère utilisé pour préparer composé polyisocyanate à motifs biuret (a) de la composition selon l'invention est choisi parmi les composés monomères diisocyanates aliphatiques linéaires tels que 2-methyl-pentane-diisocyanate (MPDI), hexamethylene-diisocyanate (HDI), tetramethylene-diisocyanate, pentamethylene-diisocyanate, octamethylene-diisocyanate, butylene-diisocyanate, octylene-diisocyanate, trimethtylhexane diisocyanate, dodécane-diisocyanate, undécane-diisocyanate, 2,2,4-tris-methyl-hexamethylene-diisocyanate, 2,4,4-tris-methyl-hexamethylene-diisocyanate, 1,8-diisocyanato-4-isocyanato-methyl-octane, 1-decane-triisocyanate. Le monomère isocyanate préféré est l'hexamethylene-diisocyanate (HDI).

De manière préférée, le composé (b) mis en oeuvre selon l'invention est majoritairement issu d'un monoalcool, notamment d'un monoalcool en C₁-C₂₀, et d'un monomère isocyanate.

De manière plus préférée, le composé (b) mis en oeuvre selon l'invention est majoritairement issu du mélange d'un monoalcool et de polyols ou d'un monoalcool et de diols.

De manière avantageuse selon l'invention, le composé (a) et le composé (b) peuvent être préparés au moyen du même composé monomère isocyanate.

Pour connaître la viscosité du composé (b), il est possible de réaliser la synthèse de manière isolée, éventuellement en présence d'un catalyseur. Les différentes synthèses possibles sont connues de l'homme de l'art.

De manière préférée, le composé (b) est un composé de formule (I) 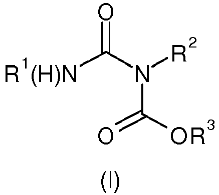 dans laquelle
▪ R¹ et R², identiques ou différents, représentent indépendamment un groupement C₂-C₂₀ alkyl, linéaire, cyclique ou ramifié, comprenant au moins une fonction isocyanate ; de préférence un groupement C₂-C₁₂ alkyl, linéaire, cyclique ou ramifié, comprenant au moins une fonction isocyanate ; plus préférentiellement un groupement C₂-C₁₂ alkyl, linéaire ou ramifié, comprenant au moins une fonction isocyanate ; en particulier un groupement C₄-C₁₂ alkyl, linéaire ou ramifié, comprenant au moins une fonction isocyanate ;
▪ R³ représente indépendamment un groupement C₅-C₁₀ hétérocycloalkyl ; un groupement C₅-C₁₀ aromatique ; un groupement C₅-C₁₀ alkyl-aryl; un groupement C₁-C₂₀ alkyl, linéaire, cyclique ou ramifié ; de préférence un groupement C₁-C₁₂ alkyl, linéaire, cyclique ou ramifié ; plus préférentiellement un groupement C₁-C₁₂ alkyl, linéaire ou ramifié ; notamment un groupement C₁-C₁₂ alkyl, linéaire ou ramifié ; en particulier un groupement C₃-C₈ alkyl, linéaire ou ramifié.

Au sein des groupements R¹ et R² des composés de formule (I) la fonction isocyanate peut être une fonction terminale et donc se trouver à l'extrémité de la chaîne alkyl ou bien être une ramification de cette chaîne alkyl. De manière préférée, les groupements alkyl ne comprennent pas d'atome de carbone tertiaire.

Comme composés de formule (I), on préfère les composés pour lesquels R¹ et R², identiques ou différents, représentent indépendamment un groupement C₂-C₈ alkyl, linéaire ou ramifié, comprenant une fonction isocyanate ; de préférence un groupement hexyl comprenant une fonction isocyanate.

Comme composés de formule (I), on préfère les composés pour lesquels R³ représente indépendamment un groupement C₃-C₈ alkyl, linéaire ou ramifié ; de préférence un groupement choisi parmi propyl, butyl, hexyl, octyl, 2-ethyl-hexyl. Les différents isomères de ces groupements conviennent également.

Pour les composés de formule (I), R³ peut également représenter un groupement C₅-C₁₀ hétérocycloalkyl comprenant au moins un hétéroatome choisi parmi O, S et N.

La fonctionnalité NCO des composés de formule (I), selon l'invention peut varier autour de la valeur de 2, notamment selon les conditions particulières de préparation de ces composés.

De manière avantageuse selon l'invention, le composé polyisocyanate à motifs biuret (a) et au moins un composé diluant réactif polaire, protique (b) peuvent être préparés au moyen du même composé monomère isocyanate.

La composition selon l'invention peut également comprendre d'autres composés. Elle peut notamment comprendre d'autres composés à motifs allophanate, par exemple des allophanates de polyols ou de diols.

La composition selon l'invention comprend au moins un composé (d). De manière préférée, le composé (d) est porteur d'au moins une fonction isocyanate. De manière plus préférée, le composé (d) est porteur d'au moins deux fonctions isocyanates. Le composé (d) ne contient pas d'azote porteur d'hydroène capable d'établir une liaison hydrogène.

Le composé (d) selon l'invention possède avantageusement une viscosité mesurée à 25 °C inférieure à 500 mPa.s, de préférence une viscosité mesurée à 25 °C allant de 20 à 500 mPa.s. De manière plus préférée, le composé (d) selon l'invention possède une viscosité mesurée à 25 °C inférieure à 250 mPa.s, de préférence inférieure à 150 mPa.s.

Le composé (d) selon l'invention est choisi parmi un composé de formule (IV), un composé de formule (V), un composé de formule (VI) et un composé de formule (VII) 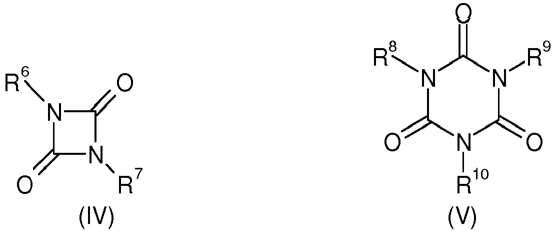 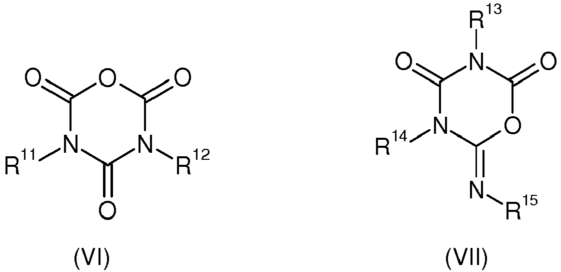 dans lesquelles
▪ R⁶ et R⁷, identiques ou différents, représentent indépendamment un groupement C₂-C₂₀ alkyl, linéaire, cyclique ou ramifié, comprenant au moins une fonction isocyanate ; de préférence un groupement C₂-C₁₂ alkyl, linéaire, cyclique ou ramifié, comprenant au moins une fonction isocyanate ; plus préférentiellement un groupement C₂-C₁₂ alkyl, linéaire ou ramifié, comprenant au moins une fonction isocyanate ; en particulier un groupement C₄-C₁₂ alkyl, linéaire ou ramifié, comprenant au moins une fonction isocyanate ;
▪ R⁸, R⁹, R¹¹, R¹², R¹³ R¹⁴ et R¹⁵, identiques ou différents, représentent indépendamment un groupement C₂-C₂₀ alkyl, linéaire, cyclique ou ramifié ; de préférence un groupement C₂-C₁₂ alkyl, linéaire, cyclique ou ramifié ; plus préférentiellement un groupement C₂-C₁₂ alkyl, linéaire ou ramifié ; en particulier un groupement C₄-C₁₂ alkyl, linéaire ou ramifié ; un groupement C₂-C₂₀ alkyl, linéaire, cyclique ou ramifié, comprenant au moins une fonction isocyanate ; de préférence un groupement C₂-C₁₂ alkyl, linéaire, cyclique ou ramifié, comprenant au moins une fonction isocyanate ; plus préférentiellement un groupement C₂-C₁₂ alkyl, linéaire ou ramifié, comprenant au moins une fonction isocyanate ; en particulier un groupement C₄-C₁₂ alkyl, linéaire ou ramifié, comprenant au moins une fonction isocyanate.

Les composés polyisocyanates de la composition selon l'invention peuvent être utilisés comme durcisseurs polyisocyanates en association avec des composés comprenant des atomes d'hydrogène mobiles tels que polyols, polyamines et polythiols. La composition selon l'invention peut alors être utilisée pour réaliser des revêtements ou des adhésifs comprenant une ou plusieurs fonctions polyuréthanes, polyurées, polythiouréthanes, polyamides. La composition selon l'invention peut également être utilisée pour réaliser des revêtements issus de la réaction des fonctions isocyanates avec les composés comprenant des atomes d'hydrogène mobiles par la création d'une liaison uréthane, allophanate, urée, biuret, isocyanurate, acylurée, amide.

La composition selon l'invention peut également être utilisée comme composition polyisocyanate de départ pour une transformation en composés réticulables, notamment des composés réticulables par rayonnement actinique, en particulier par rayonnement UV. La composition selon l'invention peut ainsi être utilisée pour former des composés dérivés contenant une liaison double capable d'une polymérisation avec une autre liaison double. On peut ainsi citer à titre d'exemples les dérivés uréthanes acrylates, uréthanes méthacrylates, uréthanes itaconates, les dérivés urées, amides, allophanates, acylurées acrylates, acylurées méthacrylates. Ces dérivés peuvent mis en réticulation avec d'autres composés à double liaison activés de type ester éther capables d'être co-polymérisés avec les dérivés à double liaison selon l'invention. Ces produits sont par exemple issus de la réaction des compositions de l'invention avec les dérivés hydroxyéthyle acrylate, hydroxypropyle acrylate, hydroxyéthyle méthacrylate, hydroxypropyle méthacrylate, de l'acide acrylique, de l'acide méthacrylique, des composés hydroxy alkyle acrylate, des composés hydroxy alkyle méthacrylate comprenant des liaisons éthers issues par exemples des chaînes comprenant des motifs oligo éthylène glycol , des motifs oligo propylène glycol ou des motifs oligo tétraméthylène glycol.

La composition selon l'invention peut également être utilisée sous la forme de composés à fonctions isocyanates masquées. Ces composés à fonctions isocyanates masquées peuvent être associés à des composés comprenant des atomes d'hydrogène mobiles, notamment présents au sein de fonctions OH, NH₂, -NH-, SH, C(O)OH. On peut alors former des formulations dites à un seul composant qui sont stables à température ambiante pendant plusieurs jours, voire plusieurs semaines et mêmes plusieurs mois. Par une augmentation de la température, on peut former un film de revêtement du fait de la restauration thermique des fonctions isocyanates par la libération des agents masquants. La température de réticulation et la stabilité à température ambiante des formulations 1K obtenues à partir de la composition selon l'invention peut varier en fonction de l'agent de masquage utilisé. Les agents masquants sont généralement connus de l'homme du métier. Ainsi les agents masquants de type imidazole ou de type imidazole substitué sur le noyau par différents groupes alkyles pourront être réticulés à basse température de l'ordre de 80 °C. Des agents masquants de type caprolactame donneront des stabilités au stockage plus élevées, de l'ordre de plusieurs mois à température ambiante ; une réticulation efficace doit alors généralement être conduite à une température supérieure à 150 °C. Comme autres agents masquants, on peut citer les oximes tels que la méthyle éthyle cétoxime, la cyclohexanoneoxime, les dérivés du pyrazole ou des alkyles pyrazoles tels que le diméthyle pyrazole, les esters d'alkyle de l'acide malonique ou les bétacéto esters, les amines encombrées telles que la diisopropylamine ou la tert butyl benzylamine, les dérivés des phénols ou des esters des acides hydroxybenzoiques ou salicyliques.

La composition selon l'invention peut également être utilisée pour préparer des formulations hydrodispersables par greffage d'un composé polyéther, de préférence mono alkyle éther. De préférence, ce composé polyéther ne comprend qu'une fonction réactive avec la fonction isocyanate. On peut également utiliser un sel d'amine, de préférence un sel d'amine tertiaire, ou encore un sel d'aminoalkyle sulfonique ou un sel de sulfamate. On peut encore procéder par simple ajout de composés à base de dialkyles esters phosphates, de monoalkyles esters phosphates, de sulfonates neutralisés par des amines, de préférence par des amines tertiaires.

Après transformation, ces composés peuvent être utilisés avec des polyols hydrodispersables ou avec des polyuréthanes dispersés en phase aqueuse pour conduire à des peintures ou à des vernis pour phase aqueuse. Ils peuvent également être utilisés pour préparer des revêtements ou des adhésifs issus de formulations aqueuses.

La composition selon l'invention peut également être utilisée pour préparer des dispersions de polyisocyanates masqués en phase aqueuses ou pour préparer des dispersions de formulations 1 K.

La composition selon l'invention peut encore être mise en réaction avec des composés de type alkoxysilanes tels que les aminopropyle (di ou tri) alkoxysilanes, les aminométhyle (di ou tri) alkoxysilanes, les aminopropyle silazanes ou les aminométhyle silazanes, pour donner des systèmes réticulables par l'humidité tels que les MS polymères. Elle peut également donner des systèmes qui par réaction avec d'autres composés de type alkoxysilane pourront permettre d'améliorer certaines propriétés mécaniques telle que la résistance à la rayure.

La composition selon l'invention peut être utilisée dans de nombreuses applications qui mettent en jeu des compositions polyisocyanates.

Est également décrite une composition pour revêtement polyuréthane ou une composition pour revêtement polyurée qui comprend une composition selon l'invention et au moins un composé comprenant un atome d'hydrogène pouvant réagir avec un groupement isocyanate. La composition de revêtement comprend donc au moins une composition selon l'invention et au moins un composé comprenant au moins un groupement portant un atome d'hydrogène réactif avec une fonction isocyanate, de préférence choisi parmi les polyols, les amines. De manière avantageuse, la composition selon l'invention permet une vitesse de formation accrue du revêtement par rapport à une composition de durcisseur de l'état de la technique.

Est également décrite une composition pour adhésif polyuréthane ou une composition pour adhésif polyurée comprenant au moins une composition selon l'invention et au moins un composé comprenant un atome d'hydrogène pouvant réagir avec un groupement isocyanate.

Lors de la mise en oeuvre de la composition selon l'invention, tout ou partie des fonctions NCO de la composition selon l'invention peuvent être protégées. Elles peuvent également être bloquées en tout ou partie.

Est également décrite une composition hydrodispersable comprenant une composition selon l'invention et au moins un agent tensio-actif ionique ou au moins un agent tensio-actif non-ionique. Est également décrite une composition hydrodispersable comprenant une composition selon l'invention dont les fonctions NCO sont totalement ou partiellement substituées au moyen d'au moins un agent tensio-actif ionique ou au moyen d'au moins un agent tensio-actif non-ionique.

La composition selon l'invention est également utilisée de manière avantageuse pour la préparation de matériaux polyuréthane, de matériaux polythiouréthane, de matériaux polyurée ou de matériaux polyamide. La composition selon l'invention peut alors être combinée à des composés comprenant des atomes d'hydrogène mobiles pour préparer de tels, notamment des matériaux en masse, en particulier des matériaux pour la préparation d'objets dont l'épaisseur constitue une propriété essentielle. La composition selon l'invention est également avantageuse lors de sa mise en oeuvre par moulage par injection réactive (RIM ou reacting injecting molding).

L'invention concerne également l'utilisation d'une composition selon l'invention dans les domaines de la chimie organique en général, des durcisseurs, des mastics, des adhésifs, des compositions réticulables par irradiation UV, des compositions réticulables thermiquement pour les compositions selon l'invention dans lesquelles les fonctions isocyanates sont bloquées. L'invention concerne préférentiellement l'utilisation d'au moins une composition selon l'invention comme durcisseur pour la préparation d'un revêtement ou d'un adhésif. De manière préférée, ces utilisations concernent la préparation d'un revêtement polyuréthane, d'un revêtement polyurée, d'un revêtement poly(urée-uréthane) ou la préparation d'un adhésif polyuréthane, d'un adhésif polyurée, d'un adhésif poly(urée-uréthane).

Un autre objet de l'invention concerne un procédé de préparation d'une composition selon l'invention. L'invention fournit donc un procédé de préparation d'une composition dont la fonctionnalité isocyanate moyenne est supérieure à 2,5, et dont le titre NCO est compris entre 10 et 25 % en poids, comprenant
- au moins un composé polyisocyanate à motifs biuret (a) ;
- au moins un composé diluant réactif polaire, protique (b), choisi parmi les composés polyisocyanates à motifs allophanate, dont la viscosité mesurée à 25 °C est inférieure à 500 mPa.s ;
- au moins un composé d'addition (c) d'un composé (a) et d'un composé (b) et
- au moins un composé diluant réactif aprotique (d) choisi parmi un composé de formule (IV), un composé de formule (V), un composé de formule (VI) et un composé de formule (VII) tels que définis plus haut.

Ce procédé comprend les étapes suivantes:
1) préparation en présence d'un catalyseur unique choisi parmi les carboxylates de zinc et les alkylcarboxylates de zinc, d'au moins un composé polyisocyanate à motifs biuret (a);
2) préparation en présence d'un catalyseur unique choisi parmi les carboxylates de zinc et les alkylcarboxylates de zinc, d'au moins un composé diluant réactif polaire, protique (b) choisi parmi les composés polyisocyanates à motifs allophanate, dont la viscosité mesurée à 25 °C est inférieure à 500 mPa.s;
3) préparation d'au moins un composé d'addition (c);
4) préparation d'au moins un composé diluant réactif polaire, protique (d); puis
5) séparation du monomère isocyanate en excès.

Selon la nature des composés (a), (b) et (c), le procédé de préparation peut varier. Ainsi de manière avantageuse l'étape 1), 2) selon le procédé de préparation peut être réalisée en premier. De manière avantageuse, l'étape 1), 2) ou 4) selon le procédé de préparation peut être réalisée en premier. De manière particulièrement avantageuse, les étapes 1) et 2) ou 2) et 3) selon le procédé de préparation peuvent être réalisées simultanément. De manière particulièrement avantageuse, les étapes 1) et 2) ou 2) et 3) ou 3) et 4) ou 1) et 2) et 3) et 4) selon le procédé de préparation peuvent être réalisées simultanément.

Lors de la mise en oeuvre du procédé selon l'invention, les monomères utilisés pour préparer le composé (a) et le monomère utilisé pour préparer le composé (b) sont choisis indépendamment. De manière préférée, les composés (a) et (b) sont préparés à partir du même monomère, en particulier choisi parmi les monomères diisocyanates aliphatiques linéaires et notamment l'hexamethylene-diisocyanate (HDI).

Selon un procédé préféré, il est possible d'utiliser un seul catalyseur pour faire la réaction d'allophanatation (étape 2 de préparation du composé (b)) et la réaction de biurétisation (étape 1 de préparation du composé (a)).

De manière préférée, le ratio molaires des catalyseurs ou du catalyseur unique est un ratio déterminé de la manière suivante : somme des catalyseurs / nombre de fonctions OH (H₂O + Alcools). Ce ratio est compris entre 1.10⁻² et 1.10⁻⁵. Lorsque le catalyseur compte uniquement des composés organo métalliques alors ce ratio molaire somme des catalyseurs / nombre de fonctions hydroxyles est équivalent au ratio molaire Métal / somme des fonctions OH (H2O + ALCOOLS).

De manière également préférée, le procédé selon l'invention est un procédé continu.

De manière avantageuse, le composé (d) de formule (IV) ou (V) peut être préparé lors d'une étape finale du procédé selon l'invention, par chauffage du milieu réactionnel alors que les autres réactions sont achevées.

D'autres composés peuvent être ajoutés à la composition selon l'invention comme au moins un pyrophosphate ou un de ses dérivés, un diphosphate ou un de ses dérivés et des sels et esters de l'acide pyrophosphorique.

Les exemples qui suivent fournissent une illustration des différents aspects de l'invention.

Les produits TOLONATES utilisés dans les exemples d'applications sont des polyisocyanates commerciaux vendus par la société Vencorex. Le TOLONATE HDT a une viscosité à 25°C de 2 400 +/- 400 mPas et un titre NCO de 22 +/-1 % poids. Le TOLONATE HDT LV a une viscosité à 25°C de 1 200 +/- 300 mPas et un titre NCO de 23 +/-1 % poids. Le TOLONATE HDB LV a une viscosité à 25°C de 2 000 +/- 500 mPas et un titre NCO de 23,5 +/-1 % poids

Le polyol ALBODUR U 955 est un polyol vendu par la société Alberding et Boley.

Les polyols SETAL et SETALUX sont des polyols vendus par la société Nuplex resins.

Les catalyseurs organo-métalliques sont des catalyseurs fournis par les sociétés Aldrich, Acros, STREM, Alfa Aesar ou TCI.

Les composés esters di alkyles phosphates sont des produits fournis par la société Acros. La méthyle amyle cétone (MAK) est fournie par la société Alfa Aesar.

Le solvesso 100 est fourni par la société Exxon Mobil.

Pour tous les essais de synthèse, on procède à une filtration du milieu réactionnel avant distillation afin d'éliminer les insolubles éventuellement formés. Le filtre utilisé est un filtre Millipore PVDF Durapor de 0,45 micron. Le taux d'insolubles est défini par pesée du filtre avant et après filtration

Les méthodes d'analyse utilisées sont connues en tant que telles de l'homme du métier.

L'analyse des compositions en oligomères isocyanates a été réalisée par chromatographie de perméation de gel couplée à un analyseur infrarouge. Pour réaliser l'analyse, on injecte une quantité connue d'échantillon de composition de polyisocyanates sur un ensemble de 2 colonnes PL GEL en série. Les oligomères sont élués avec du dichlorométhane stabilisé avec de l'amylène selon leur taille, poids et structure moléculaire. On utilise comme étalon interne le benzonitrile à raison de 100 microlitre pour 10 mL de dichlorométhane. Les colonnes de PL Gel utilisées sont la PL GEL 50 A° 5 microns d'une longueur de 60 cm et d'un diamètre de 7,5 mm et la PL Gel 100 A° 5 microns d'une longueur de 60 cm et d'un diamètre de 7,5 mm. La pression de colonne est de l'ordre de 81 bars. Le débit est de 1 mL/mn. En sortie de colonnes, les composés élués sont analysés par spectroscopie infrarouge et quantifiés.

Les composés (a) contiennent les structures polyisocyanates biuret issues de 3 unités monomères notées biuret (n = 3) contenant une seule fonction biuret, les structures polyisocyanates biuret issues de 5 unités monomères notées biuret (n=5) contenant deux fonctions biurets, les oligomères polyisocyanates biurets issues de plus de 5 unités monomère notées biuret (n>5) et les composés contenant dans leur structure au moins une fonction biuret et au moins une fonction uretidine dione.

La quantité des oligomères de la composition est donnée en quantité pondérale sauf pour les composés (c) pour lesquels on donne une quantité molaire de fonctions allophanates et les fonctions biurets contenues dans les composés (c). Le calcul de la fraction molaire de ces fonctions est obtenue en soustrayant les quantités molaires des composés (b) et du ou des intermédiaires des composés (b) contenues dans la masse de composition finale à la quantité molaire du ou des alcool(s) introduit(s) dans le milieu réactionnel. Le calcul est précisé dans les exemples.

Dans les exemples suivants le ou les catalyseurs ne comprennent qu'un seul atome de métal dans leur structure donc le ratio molaire somme des catalyseurs / nombre de fonctions hydroxyles est équivalent au ratio molaire Métal / somme des fonctions OH (H2O + ALCOOLS).

### Exemple 1 : préparation d'une composition polvisocyanate de basse viscosité avec un catalyseur unique

Dans cet essai, le bis-2-éthyl hexanoate de zinc est utilisé comme catalyseur unique de synthèse du composé (b) et des composés (a).

Dans un réacteur tricol double enveloppe, équipé d'une agitation mécanique, d'un réfrigérant et d'une ampoule d'addition, on ajoute, sous atmosphère inerte, à température ambiante, 600 g (3,571 mol) d'hexaméthylène diisocyanate (HDI). La température du milieu réactionnel est portée à 110 °C. 0,13 g de bis-(2 éthyl hexanoate) de zinc à 80 % en poids dans le white spirit est ajouté dans 4,26 g (57,6 m mol) de 1butanol. Cette formulation de catalyseur est ajoutée au milieu réactionnel. La température du milieu réactionnel est élevée à 130 °C et maintenue pendant encore 1 heure. Le titre NCO du milieu réactionnel est de l'ordre de 1,15 mol pour 100 g. On constate par analyse infra-rouge que le diluant réactif allophanate de HDI et de n butyle est formé. 6,6 g d'eau liquide sont ensuite ajoutés au milieu réactionnel en 1 heure. Après ajout de l'eau, la température du milieu réactionnel est élevée à 150°C. Après 2 heures de réaction à 150°C, la réaction est arrêtée par ajout de 0,670 g de dibutyle phosphate acide (3,18 mmol). Le titre NCO de la masse réactionnelle avant distillation est de 0,980 mol pour 100 g de milieu réactionnel. 559,6 g de masse réactionnelle sont filtrés pour éliminer 0,37 g d'impuretés solides. Le filtrat est ensuite distillé sous vide à 140 °C pour éliminer le monomère HDI en excès. On obtient 169 g de composition polyisocyanate. Le rendement pondéral récupéré est de 30,2 %.

Les caractéristiques des produits de l'exemple 1 obtenu après distillation du monomère diisocyanate utilisé en excès sont présentées dans le tableau 1. La fonctionnalité molaire moyenne en NCO est calculée à partir de la composition analysée par chromatographie par perméation de gel GPC - IR.

**Tableau 1**

| Titre NCO % poids | Viscosité mPas 25°C | Fonctionnalité molaire moyenne en NCO | Taux de transformation de HDI en % poids |
|---|---|---|---|
| 22,9 | 1066 | 3,01 | 32,5 |

La composition polyisocyanate est analysée par chromatographie par perméation de gel couplée à une détection par infra-rouge. Elle contient des oligomères de structure biuret, un diluant réactif polaire protique de type allophanate, au moins un composé de réaction contenant une structure allophanate liée de manière covalente à une structure biuret et un diluant réactif aprotique de type uretidine dione. La répartition des composés est présentée dans le tableau 2.

**Tableau 2**

| Type de composé | Composé de la composition de l'exemple 1 | % poids |
|---|---|---|
| | HDI monomère | 0,25 |
| | Carbamate de HDI et de butyle | 0,2 |
| (d) | Uretidine dione de HDI (HDI Dimère) | 8 |
| (b) | Allophanate de HDI et de n butyle | 7,9 |
| (a) | Dimère-biuret, Biuret (n=3, n=5, n> 5) | 74,4 |
| (c) | Dérivés polyisocyanates biuret- allophanate de HDI et de n butyle | 8,95 |

Dans le cas de l'exemple 1 la fraction molaire de fonctions allophanates des composés (c) est de 0,024 pour 169 g de composition.

Elle est calculée comme suit :
Nombre de moles de composé alcool (butanol) utilisées - {Nombre de moles de composés (b) (allophanate de HDI et de n butyle) obtenues dans la masse totale de la composition polyisocyanate de l'exemple 1 + nombre de moles de composés intermédiaires de (b) (Carbamate de butyle et de HDI) obtenues dans la masse totale de la composition polyisocyanate de l'exemple 1} = 0,0576 - { ((7,9 g/ 410)*169 /100) + ((0,2 /242)* 169/100)} = 0,024

### Exemple 2: préparation d'une composition polyisocyanate de basse viscosité base biuret avec un autre catalyseur unique

On procède comme pour l'exemple 1 mais en utilisant le bis-néodécanoate de zinc (bNZ) comme catalyseur de la réaction en lieu et place du bis-2-éthyl hexanoate de zinc. Le bis-néodécanoate de zinc présente un meilleur profil de toxicité. Les quantités de réactifs utilisés sont présentées dans le tableau 3.

**Tableau 3**

| Réactif | HDI | Eau | 1-butanol | bNZ (extrait sec) |
|---|---|---|---|---|
| Quantité en g | 600 | 6,6 | 4,26 | 0,15 |

Les ratios molaires sont présentés dans le tableau 4.

**Tableau 4**

| NCO / OH | NCO / eau | bNZ/ NCO | bNZ / OH alcool |
|---|---|---|---|
| alcool | | | |
| 124 | 19,5 | 52.10⁻⁶ | 0,0064 |

La quantité de polyisocyanate récupérée après réaction et distillation du monomère diisocyanate est de 176 g correspondant à un rendement pondéral récupéré de 31,5 %. La quantité d'insolubles présents avant distillation est de 0,35 g sur une masse réactionnelle engagée en distillation de 562 g. Le titre NCO du milieu réactionnel avant distillation est de 0,985 mol pour 100 g.

Les caractéristiques des produits obtenus après distillation du monomère diisocyanate utilisé en excès sont présentées dans le tableau 5. La fonctionnalité molaire moyenne en NCO est calculée à partir de la composition analysée par chromatographie par perméation de gel GPC - IR.

**Tableau 5**

| Titre NCO % poids | Viscosité mPas 25°C | Fonctionnalité molaire moyenne en NCO | Taux de transformation de HDI en % poids |
|---|---|---|---|
| 22,9 | 1070 | 2,99 | 33,4 |

La répartition des composés dans la composition finale est présentée dans le tableau 6.

**Tableau 6**

| Type de composé | Composé de la composition de l'exemple 2 | % poids |
|---|---|---|
| | HDI monomère | 0,15 |
| | Carbamate de HDI et de butyle et de HDI et d'isopropyle | 0,2 |
| (d) | Uretidine dione de HDI (HDI Dimère) | 8 |
| (b) | Allophanate de HDI et de n butyle | 7,9 |
| (a) | Dimère-biuret, Biuret (n=3, n=5, n> 5) | 76,1 |
| (c) | Dérivés polyisocyanates biuret- allophanate de HDI et de n butyle | 7,65 |

Dans le cas de l'exemple 2 la fraction molaire de fonctions allophanates des composés (c) est de 0,022 pour 176 g de composition. On procède au même type de calcul que pour l'exemple 1.

### Exemple 3: préparation d'une composition polyisocyanate de basse viscosité avec des conditions opératoires modifiées

On procède comme pour l'exemple 2 ; les quantités de réactifs sont les mêmes mais les conditions opératoires sont modifiées. La formulation alcoolique de catalyseur est ajoutée à température ambiante au milieu réactionnel contenant le HDI. La température du milieu réactionnel passe de la température ambiante à 150°C en 1 heure. L'eau est injectée en 1 heure dès que la température a atteint 130°C. Le milieu réactionnel est maintenu à 150°C 2 heures après injection de la totalité de l'eau.

Dans ces conditions le titre NCO de la masse réactionnelle est de 1,165 mol de NCO pour 100 g avant le début de l'injection de l'eau. Le titre NCO du milieu réactionnel avant distillation est de 0,978 mol pour 100 g. Le taux de transformation du HDI est de 32 % en poids. 454 g de milieu réactionnel sont filtrés sur millipore puis purifiés par distillation du monomère HDI. La quantité d'insolubles obtenus par filtration de la masse réactionnelle avant distillation est de 0,54 g. Après distillation, on récupère 149 g de polyisocyanate à motifs biuret de basse viscosité, soit un rendement pondéral récupéré de 33 %. Les caractéristiques des produits obtenus après distillation sont présentées dans le tableau 7.

**Tableau 7**

| Titre NCO % poids | Viscosité mPas 25°C | Taux de transformation de HDI en % |
|---|---|---|
| 22,72 | 1 371 | 32 |

La répartition des composés de la composition finale est présentée dans le tableau 8.

**Tableau 8**

| Type de composé | Composé de la composition de l'exemple 3 | % poids |
|---|---|---|
| | HDI monomère | 0,2 |
| | Urée de HDI | 0,1 |
| | Carbamate de HDI et n-butyle | 0,2 |
| (d) | Uretidine dione de HDI (HDI Dimère) | 7,1 |
| (b) | Allophanate de HDI et de n butyle | 9,7 |
| (a) | Dimère-biuret, Biuret (n=3, n=5, n> 5) | 73.9 |
| (c) | Dérivés polyisocyanates biuret- allophanate de HDI et de n butyle | 9 |

Dans le cas de l'exemple 3 la fraction molaire de fonctions allophanates des composés (c) est de 0,022 pour 149 g de composition. On procède au même type de calcul que pour l'exemple 1.

### Exemple 4: préparation d'une composition polyisocyanate de basse viscosité avec d'autres conditions opératoires modifiées

On procède comme pour l'exemple 3 ; les quantités de réactifs sont les mêmes mais les conditions opératoires sont modifiées. La formulation alcoolique de catalyseur est ajoutée à température ambiante au milieu réactionnel contenant le HDI. La température du milieu réactionnel passe de la température ambiante à 150°C en 1 heure. L'eau est injectée en 1 heure dès que la température a atteint 130°C une fois réalisé un palier de 1 heure à 130 °C. Le milieu réactionnel est maintenu à 150°C 3 heures après injection de la totalité de l'eau.

Dans ces conditions le titre NCO de la masse réactionnelle est de 1,154 mol de NCO pour 100 g avant le début de l'injection de l'eau. Le titre NCO du milieu réactionnel avant distillation est de 0,980 mol pour 100 g. Le taux de transformation du HDI est de 32 % en poids. 567 g de milieu réactionnel sont filtrés sur millipore puis purifiés par distillation du monomère HDI. La quantité d'insolubles obtenus par filtration de la masse réactionnelle avant distillation est de 0,44 g. Après distillation, on récupère 186 g de polyisocyanate à motifs biuret de basse viscosité, soit un rendement pondéral récupéré de 33 %. Les caractéristiques des produits obtenus après distillation sont présentées dans le tableau 9.

**Tableau 9**

| Titre NCO % poids | Viscosité mPas 25°C | Taux de transformation de HDI en % |
|---|---|---|
| 22,9 | 1 157 | 32 |

La répartition des composés de la composition finale est présentée dans le tableau 10.

**Tableau 10**

| Type de composé | Composé de la composition de l'exemple 4 | % poids |
|---|---|---|
| | HDI monomère | 0,3 |
| | Urée de HDI | 0,1 |
| | Carbamate de HDI et n-butyle | 0,1 |
| (d) | Uretidine dione de HDI (HDI Dimère) | 8,5 |
| (b) | Allophanate de HDI et de n butyle | 10,3 |
| (a) | Dimère-biuret, Biuret (n=3, n=5, n> 5) | 70 |
| (c) | Dérivés polyisocyanates biuret- allophanate de HDI et de n butyle | 10,7 |

Dans le cas de l'exemple 4 la fraction molaire de fonctions allophanates des composés (c) est de 0,020 pour 186 g de composition. On procède au même type de calcul que pour l'exemple 1.

### Exemple 5: préparation d'une composition polyisocyanate de basse viscosité avec d'autres conditions opératoires modifiées

On procède comme pour l'exemple 3; les quantités de réactifs sont les mêmes mais les conditions opératoires sont modifiées. La formulation alcoolique de catalyseur est ajoutée à température ambiante au milieu réactionnel contenant le HDI. La température du milieu réactionnel passe de la température ambiante à 150°C en 1 heure. L'eau est injectée en 1 heure dès que la température a atteint 130°C. Le milieu réactionnel est maintenu à 150°C 2 heures après injection de la totalité de l'eau.

Dans ces conditions le titre NCO de la masse réactionnelle est de 1,167 mol de NCO pour 100 g avant le début de l'injection de l'eau. Le titre NCO du milieu réactionnel avant distillation est de 0,968 mol pour 100 g. Le taux de transformation du HDI est de 34 % en poids. 597 g de milieu réactionnel sont filtrés sur millipore puis purifiés par distillation du monomère HDI. La quantité d'insolubles obtenus par filtration de la masse réactionnelle avant distillation est de 0,37 g. Après distillation, on récupère 189 g de polyisocyanate à motifs biuret de basse viscosité, soit un rendement pondéral récupéré de 33,5 %. Les caractéristiques des produits obtenus après distillation sont présentées dans le tableau 11.

**Tableau 11**

| Titre NCO % poids | Viscosité mPas 25°C | Taux de transformation de HDI en % |
|---|---|---|
| 22,6 | 1 366 | 34 |

La répartition des composés de la composition finale est présentée dans le tableau 12.

**Tableau 12**

| Type de composé | Composé de la composition de l'exemple 5 | % poids |
|---|---|---|
| | HDI monomère | 0,5 |
| | Urée de HDI | 0,2 |
| | Carbamate de HDI et n-butyle | 0,2 |
| (d) | Uretidine dione de HDI (HDI Dimère) | 8,1 |
| (b) | Allophanate de HDI et de n butyle | 7,9 |
| (a) | Dimère-biuret, Biuret (n=3, n=5, n> 5) | 72.9 |
| (c) | Dérivés polyisocyanates biuret- allophanate de HDI et de n butyle | 10,9 |

Dans le cas de l'exemple 5 la fraction molaire de fonctions allophanates des composés (c) est de 0,0212 pour 189 g de composition. On procède au même type de calcul que pour l'exemple 1.

### Exemple 6: préparation d'une composition polyisocyanate de basse viscosité avec plusieurs composés (b) et deux catalyseurs

On procède à la synthèse de deux diluants réactifs polaires, pratiques de type allophanate en utilisant 2 mono alcools. On utilise deux types de catalyseurs. Pour la synthèse des diluants réactifs allophanate, on utilise un sel métallique d'acide carboxylique, particulièrement le bis-2-éthyle hexanoate de zinc (catalyseur 1). Pour la synthèse du biuret, on utilise un dialkyle phosphate acide (catalyseur 2 ou DBP). Les quantités de réactifs utilisés sont présentées dans le tableau 13

**Tableau 13**

| Réactifs | HDI | Eau | 1-butanol | 2-propanol | Catalyseur 1 | Catalyseur 2 |
|---|---|---|---|---|---|---|
| Quantité en g | 700 | 7,7 | 4,9 | 2,1 | 0,137 | 0,875 |

Les ratios molaires sont présentés dans le tableau 14

**Tableau 14**

| NCO / OH alcools | NCO / eau | Catalyseur 1/NCO | Catalyseur 1 / OH alcools | DBP / NCO | NCO / DBP |
|---|---|---|---|---|---|
| 83 | 19,5 | 47.10⁻⁶ | 0,0039 | 0,0005 | 1984 |

Le titre NCO du milieu réactionnel en fin de réaction est de 0,957 mol pour 100 g. Après filtration, la quantité de milieu réactionnel envoyée en distillation est de 676 g. Le taux d'insolubles est négligeable. La quantité de composition polyisocyanate récupérée après distillation du monomère diisocyanate en excès est de 239 g ; ce qui donne un rendement pondéral de 35,4 %. Les caractéristiques des produits obtenus après distillation sont présentées dans le tableau 15.

**Tableau 15**

| Titre NCO % poids | Viscosité mPas 25°C | Fonctionnalité molaire moyenne en NCO | Taux de transformation de HDI en % (GPC) |
|---|---|---|---|
| 22,6 | 1202 | 3 | 39 |

La répartition est présentée dans le tableau 16.

**Tableau 16**

| Type de composé | Composé de la composition de l'exemple 6 | % poids |
|---|---|---|
| | HDI monomère | 0,21 |
| | Mono Carbamates de HDI | 0,2 |
| (d) | Uretidine dione de HDI (HDI Dimère) | 7,7 |
| (b) | Allophanates de HDI et de n butyle et de HDI et d'isopropyle (n=2) | 12,7 |
| (a) | Dimère-biuret, Biuret (n=3, n=5, n> 5) | 72.2 |
| (c) | Dérivés polyisocyanates Biuret-allophanate de HDI et de (n butyle et isopropyle) | 7,6 |

Dans le cas de l'exemple 6 la fraction molaire de fonctions allophanates des composés (c) est de 0,025 mole pour 239 g de composition. On procède au même type de calcul que pour l'exemple 1.

### Exemple 7: préparation d'une composition polyisocyanate de basse viscosité avec un ratio de catalyseur différent

On reproduit les conditions de l'exemple 6 mais avec un ratio Catalyseur 1 /NCO différent. Les quantités de réactifs utilisés sont présentées dans le tableau 17.

**Tableau 17**

| Réactif | HDI | Eau | 1-butanol | 2-propanol | Catalyseur 1 | Catalyseur 2 |
|---|---|---|---|---|---|---|
| Quantité en g | 500 | 5,5 | 3,5 | 1,5 | 0,052 | 0,625 |
| Quantité en mol | 2,976 | 0,306 | 0,047 | 0,0246 | 0,00015 | 0,003 |

Les ratios molaires sont présentés dans le tableau 18.

**Tableau 18**

| NCO / OH alcools | NCO / eau | Catalyseur 1/NCO | Catalyseur 1/ OH alcools | DBP / NCO | NCO / DBP |
|---|---|---|---|---|---|
| 83 | 19,5 | 25.10⁻⁶ | 0,0021 | 0,0005 | 1984 |

Le titre NCO du milieu réactionnel en fin de réaction est de 0,967 mol pour 100 g. Après filtration, la quantité de milieu réactionnel envoyée en distillation est de 468 g. Le taux d'insolubles est négligeable. La quantité de composition polyisocyanate récupérée après distillation du monomère diisocyanate en excès est de 163 g ; ce qui donne un rendement pondéral de 34,8 %. Les caractéristiques des produits obtenus après distillation sont présentées dans le tableau 19.

**Tableau 19**

| Titre NCO % poids | Viscosité mPas 25°C | Fonctionnalité molaire moyenne en NCO | Taux de transformation de HDI en % (GPC) |
|---|---|---|---|
| 22,9 | 1328 | 3,15 | 36,6 |

La répartition des composés est présentée dans le tableau 20.

**Tableau 20**

| Type de composé | Composé de la composition de l'exemple 7 | % poids |
|---|---|---|
| | HDI monomère | 0,13 |
| | Mono Carbamates de HDI | 0,1 |
| (d) | Uretidine dione de HDI (HDI Dimère) | 9,1 |
| (b) | Allophanates de HDI et de n butyle et de HDI et d'isopropyle | 6,3 |
| (a) | Dimère-biuret, Biuret (n=3, n=5, n> 5) | 73.3 |
| (c) | Dérivés polyisocyanates biuret- allophanate de HDI et (de n butyle et d'isopropyle) | 11,07 |

Dans le cas de l'exemple 7 la fraction molaire de fonctions allophanates des composés (c) est de 0,046 mole pour 163 g de composition. On procède au même type de calcul que pour l'exemple 1.

L'analyse RMN ¹³C en présence d'acétylacétonate de fer réalisée au moyen d'un appareil Brucker AV 500 permet d'identifier et de quantifier les fonctions constitutives de la composition polyisocyanate. La répartition molaire des fonctions identifiées dans la composition polyisocyanate est présentée dans le tableau 21.

**Tableau 21**

| Fonction identifiée exprimée en mol | |
|---|---|
| Fonction isocyanurate | 0,9 |
| Fonctions dimères (Uretidine dione) | 5,2 |
| Fonction allophanates | 5,5 |
| Fonctions biuret | 19,8 |
| Somme totale des fonctions identifiées | 31,4 |
| Ratio molaire fonctions biuret / Biuret + allophanates | 78,3 |
| Ratio molaire théorique fonctions biuret/biuret + allophanates basé sur le nombre de mol d'eau et d'alcools introduit | 81,03 |

On constate que le ratio (fonctions biuret / fonctions biuret + allophanates) dosé par RMN ¹³C est en très bonne concordance avec le ratio théorique calculé à partir des composés de départ utilisés dans le procédé puisqu'il atteint une valeur de 96,6 %, montrant ainsi la performance du procédé.

Notamment, la RMN ¹³C met en évidence la présence de fonctions de type isocyanurate en faible quantité (2,84 % de l'ensemble des fonctions identifiées) qui n'avaient pas été identifiés par l'analyse par perméation de gel couplée à l'infra-rouge. Cela signifie qu'au cours du procédé de synthèse, on a eu formation de composé isocyanurate de HDI en faible quantité.

Une analyse RMN du phosphore 31 en milieu CDCl₃ montre l'absence de dibutyle phosphate acide catalyseur utilisé lors de la synthèse du biuret mais montre la présence de composés pyrophosphates symétriques et dissymétriques, dans un rapport molaire respectivement égal à 68 / 32. La présence de pyrophosphates dissymétriques montre qu'au cours de la réaction, il y a eu hydrolyse partielle d'un groupe butyle du catalyseur dibutyle phosphate acide.

### Exemple 8: applications de compositions polyisocyanates à 100 % d'extrait sec

On prépare une formulation d'un mélange d'une partie A comprenant des composés à fonctions réactives avec les fonctions isocyanates qui sont comprises dans les composés polyisocyanates d'une partie B. Les quantités sont présentées dans le tableau 22.

Comme partie A, on utilise une résine polyol (produit ALBODUR U 955) ayant 8,79 % de fonctions hydroxyles. Comme partie B, on utilise les polyisocyanates des exemples 3, 4, 5 selon l'invention, ainsi que des polyisocyanate témoins qui sont connus (produits utilisés et caractérisés : Tolonate HDT LV - titre NCO: 22,78 %, viscosité 1 185 mPa.s à 25 °C , Tolonate HDB LV - titre NCO: 23,37 %, viscosité 2 062 mPa.s à 25 °C et Tolonate HDT-titre NCO: 21,4 %, viscosité 2 526 mPa.s à 25 °C). On travaille avec un ratio molaire fonctions NCO / OH de 1 et une quantité de 200 ppm de dibutyle-dilaurate d'étain (DBTL) comme catalyseur.

Les produits sont pesés dans un bécher de 250mL puis sont mélangés avec une pale de type hélice pendant 1 min et à une vitesse de 300 t/min. Les produits sont ensuite dégazés dans un dessiccateur sous aspiration (10 bar) pendant 5 min environ jusqu'à disparition des mousses. Le temps de démoussage est relevé. Les résultats sont présentés dans le tableau 22.

**Tableau 22**

| Partie A - résine ALBODUR U955 - g | 34 | 34 | 34 | 34 | 34 |
|---|---|---|---|---|---|
| Partie B -durcisseur isocyanate - g | exemple 3 | exemple 4 | exemple 5 | produit HDTLV | produit HDT |
| | 32,54 | 32,26 | 32,69 | 32,43 | 34,52 |
| Aspect après démoussage (5 min) | disparition des mousses | | | Demande un temps démoussage > 5 min | |

On constate que les polyisocyanates à motifs biurets de basse viscosité selon l'invention présentent un temps de démoussage plus rapide et donc présentent un temps de mise en oeuvre plus rapide que les produits connus. Il s'agit d'un avantage important découlant de la très basse viscosité des polyisocyanates selon l'invention.

Pour une première série d'évaluations de la dureté shore, la formulation est coulée dans une capsule en aluminium de 74 mm de diamètre. La quantité coulée est de 30g en moyenne pour avoir une épaisseur minimum de 6 mm requise.

Pour une deuxième série d'évaluations de la traction, la formulation est coulée sur une plaque en polyéthylène de 8 cm x 12 cm. La quantité coulée est de 27g en moyenne pour avoir une épaisseur minimum de 2 mm requise. L'ensemble des préparations est stocké en salle conditionnée à 23°C et 50 % HR puis analysé après 7 jours de stockage. Les résultats sont présentés dans les tableaux 24 (dureté shore D) et 25 (aspect après traction).

**Tableau 24**

| Partie B | Dureté shore D | | | |
|---|---|---|---|---|
| | jour 1, t₀ | jour 1 , t_{15 min} | jour 3 | jour 7 |
| exemple 3 | 35/25 | 35/25 | 45/30 | 49/32 |
| exemple 4 | 36/26 | 36/26 | 44/29 | 47/31 |
| produit HDTLV | 27/18 | 27/18 | 36/25 | 42/28 |
| produit HDT | 36/25 | 36/25 | 36/26 | 42/29 |

Les résultats de propriété mécanique obtenus à partir d'une machine à traction sont présentés dans le tableau 25.

**Tableau 25**

| Polyisocyanate | Module (MPa) | Contrainte (MPa) | Déformation (%) |
|---|---|---|---|
| exemple 3 | 6,34 | 8,72 | 157,58 |
| exemple 4 | 6,04 | 7,42 | 142,61 |
| produit HDTLV | 6,94 | 5,14 | 97,71 |
| produit HDT | 7,56 | 5,76 | 105,32 |

On constate que les polyisocyanate à motif biuret de très basse viscosité selon l'invention présentent des performances meilleures par rapport aux produits Tolonate HDT LV ET Tolonate HDT. On constate également que les polyisocyanates à motifs biurets de basse viscosité selon l'invention présentent une meilleure compatibilité avec la résine ALBODUR que les produits Tolonate HDT et HDT LV.

### Exemple 9: applications de compositions polyisocyanates selon l'invention et de référence en formulations avec solvant

La mesure de la viscosité est faite à l'aide d'un rhéomètre RHEOMAT RM 300 de marque Lamy. On introduit un échantillon de produit à caractériser dans une cuve. On introduit le module d'agitation et on met le module d'agitation en marche. L'appareil donne la valeur de la viscosité du produit à un cisaillement donné et à une température donnée, pour une durée d'une minute. Le module d'agitation est choisi en fonction du domaine de viscosité cible. Pour les compositions cibles des exemples selon l'invention, la viscosité est donnée pour une valeur à 25 °C et en général pour un gradient de cisaillement de l'ordre de 100 s⁻¹.

La mesure du titre en fonctions NCO est effectuée par dosage acide-base. Le dosage est fait à l'aide d'un titrateur 916 de marque METROHM. Un échantillon de composition polyisocyanate de masse connue est mis en réaction, sous agitation, avec une solution de dibutylamine de titre et quantité connus, à température ambiante (20 °C environ). La quantité de dibutylamine est en excès par rapport aux fonctions isocyanates. Après 1 minute, le milieu réactionnel comprenant la dibutylamine en excès est dosé à température ambiante (20 °C environ) au moyen d'une solution HCl de titre connu. La différence entre la dibutylamine introduite initialement et la dibutylamine dosée correspond à la quantité de dibutylamine qui a réagi avec les fonctions isocyanates. On a ainsi accès au titre en fonctions isocyanates de la composition polyisocyanate qui est exprimé soit en % pondéral de fonctions NCO, soit en mole de fonctions NCO pour 100 g de composition polyisocyanate.

La mesure de la dureté Shore (D) est faite au moyen d'un duromètre de marque Hildebrand.

La mesure des propriétés mécaniques est réalisée à l'aide d'une machine de traction de marque MTS. Les échantillons sont préparés par coulée de 27 g environ de formulation contenant le polyisocyanate cible sur une plaque de polyéthylène de 12 cm sur 8 cm afin d'avoir une épaisseur de 2 mm. La réticulation est opérée en salle conditionnée à 23 °C et 50 % d'humidité relative (HR) et mesurée après 7 jours de stockage. On découpe ensuite à l'aide d'un emporte-pièce des haltères qui sont insérées dans les mors de la machine de traction pour opérer les mesures de propriétés mécaniques. On mesure ainsi l'allongement à la rupture exprimé en %, qui est la longueur d'allongement au-delà duquel il y a rupture du matériau, la contrainte à la rupture exprimée en N/mm² ou MPa qui est la force nécessaire pour rompre une éprouvette de section I*e. En général, plus un matériau est dur et plus la contrainte à la rupture est élevée.

Le pot-life définit le temps de vie d'une formulation de 2 composés (2K) qui sont un durcisseur polyisocyanate et un polyol ou une polyamine.

La dureté Persoz permet de mesurer la dureté d'un film de peinture après réticulation.

La résistance aux chocs est mesurée à l'aide de 2 appareils de marque Erichsen Type 304 selon les normes ISO 6272 de 1993 et ASTM D 2794 de 1984.

Pour la mesure de résistance au pliage on utilise un mandrin conique de marque Erichsen. La méthode permet de mesurer l'élasticité et la force d'adhérence d'un film de peinture sollicité en flexion.

On prépare une formulation d'un mélange d'une partie A comprenant des composés à fonctions réactives avec les fonctions isocyanates qui sont comprises dans les composés polyisocyanates d'une partie B.

Comme partie A, on utilise une formulation de polyol acrylique en solvant et comprenant un mélange de 2 polyols acryliques (produit SETALUX 1907 BA 75 X et produit Setal 1603 BA 78 X). On ajoute des additifs et un catalyseur à base de dibutyl dilaurate d'étain (DBTL) en formulation à 1% poids dans l'acétate de n-butyle (produit de la société Alfa Aesar). Les produits et quantités de la partie A sont présentées dans le tableau 26.

Les 2 résines sont pesées dans un bécher puis mises sous agitation au dispermat à pale défloculeuse, à une vitesse de 1 500 tours/min. Sous agitation, nous ajoutons un à un les produits en laissant 5 min de dispersion entre chaque ajout. À la fin des ajouts nous laissons la partie A sous agitation encore 20 min. Nous versons la formulation dans un flacon en verre fermé et la laissons dégazer à la pression atmosphérique ambiante au moins une nuit avant utilisation.

**Tableau 26**

| | % poids |
|---|---|
| Acrylique polyol (produit Setalux 1907 BA-75) | 70,06 |
| Polyester polyol (produit Setal 1603 BA-78) | 7,65 |
| Solvant 1 Méthyle amyle cétone (produit MAK) | 10,69 |
| Solvant 2 (produit Solvesso 100) | 4,37 |
| Additif 1 (produit Tinuvin 1130 - BASF) | 1,36 |
| Additif 2 (produit Tinuvin 292 - BASF) | 0,45 |
| Agent d'écoulement (produits BYK-315, BYK-332 (10% dans AcBu), BYK-358) | 0,11, |
| | 0,50, |
| | 0,30 |
| Catalyseur (dbtl 1% dans acbu) | 4,52 |

La Partie B est constituée par le durcisseur polyisocyanate. On utilise les polyisocyanates selon l'invention des exemples 4 et 5 et des systèmes polyisocyanates de référence (produits Tolonate HDT, Tolonate HDB LV ET Tolonate HDT LV - société Vencorex).

La partie C est constituée d'un mélange de solvants pour ajuster la viscosité (mise à la coupe) selon les proportions du tableau 27. Les 2 solvants sont pesés dans un flacon verre et sont mélangés durant au moins 30 min au roule-pot.

**Tableau 27**

| Solvant pour la mise à la coupe | % poids |
|---|---|
| Méthyle amyle cétone | 47,87 |
| Acétate de n butyle | 52,13 |

Ensuite, la préparation du vernis 2K est faite selon un ratio molaire des fonctions NCO/OH de 1,1. Les parties A et B sont pesées, puis mélangées à la main avec une spatule durant 3 à 5 min jusqu'à obtenir un mélange homogène. La partie solvant est ajoutée au mélange qui est homogénéisé. La quantité de solvant à ajouter a été déterminée par une mise à la coupe DIN 4 à 24 secondes environ (réajustement du solvant selon DIN 4 de 22 à 26 s). Les produits et proportions sont présentés dans le tableau 28.

**Tableau 28**

| Durcisseur isocyanate | Exemple 4 | Exemple 5 | Tolonate HDTLV | Tolonate HDBLV | Tolonate HDT |
|---|---|---|---|---|---|
| titre NCO (%) | 22,9 | 22,6 | 22,8 | 23,38 | 21,6 |
| Partie A (% en poids) | 100 | 100 | 100 | 100 | 100 |
| Partie B (% en poids) | 32,12 | 32,54 | 32,26 | 31,46 | 34,05 |
| Partie C (% en poids) | 21,57 | 22,33 | 22,25 | 22,30 | 22,87 |
| DIN 4 (secondes) | 23,21 | 24,11 | 22 | 23,70 | 23,89 |

Le film est appliqué en fonction des tests à réaliser au moyen d'un applicateur manuel ou d'un pistolet. Les films sont ensuite séchés à la température ambiante (23°C) à 50 % d'humidité relative (HR) ou cuits 30 minutes à 60°C après un flash off de 10 minutes. L'ensemble des films est stocké en salle conditionnée à 23°C et à 50 % HR.

Les duretés, brillances et résistances chimiques sont évaluées pour les films appliqués sur plaque de verre à l'aide d'un tire-film d'une épaisseur de 200 µm. Les tests de pliage et de QUV sont réalisés sur des films appliqués sur plaque d'aluminium, les revêtements étant constitués d'un primaire et d'une base. Les tests de résistance aux chocs et d'adhérence ont été réalisés sur plaque en acier, les revêtements étant constitués d'un primaire et d'une base.

**Tableau 29**

| Dureté Persoz après séchage à 70°C | Temps (jour) | | |
|---|---|---|---|
| | 1 | 3 | 8 |
| Durcisseur isocyanate | | | |
| Exemple 4 | 150 | 249 | 274 |
| Exemple 5 | 155 | 258 | 280 |
| Tolonate HDTLV | 132 | 203 | 261 |
| Tolonate HDT | 132 | 241 | 273 |

Les durcisseurs polyisocyanates de basse viscosité selon l'invention présentent un temps de construction de réseau plus court que les polyisocyanates de référence à base isocyanurate (HDT et HDTLV) (tableau 29).

Les résultats présentés dans le tableau 30 sont basés sur plusieurs cotations. La cotation pour l'adhérence va de 0 (excellent) à 5 (mauvais), la cotation pour la résistance au pliage va de 0 (excellent) à 5 (mauvais). La cotation AFNOR maximale pour la résistance aux chocs est de 100. La cotation ASTM maximale pour la résistance aux chocs est de 80. La résistance aux chocs est la meilleure lorsque la valeur de la cotation est la plus élevée.

**Tableau 30**

| Durcisseur isocyanate | Exemple | | Tolonate | |
|---|---|---|---|---|
| | 4 | 5 | HDBLV | HDT |
| Adhérence | 3 | 4 | 5 | 5 |
| Résistance au pliage | Validé | Validé | Validé | Validé |
| Résistance au choc ASTM | 80 | 80 | 70 | 80 |
| Résistance au choc AFNOR | 100 | 100 | 90 | 100 |

Les systèmes durcisseurs polyisocyanates objets de l'invention présentent une meilleure adhérence et une meilleure résistance aux chocs que le système références HDT et HDBLV.

Dans l'ensemble, les durcisseurs selon l'invention présentent une viscosité plus basse avec des performances au moins équivalentes voire meilleure que les systèmes isocyanates de référence. On note en particulier une meilleure adhérence et une meilleure résistance aux chocs.

### Exemple 10 : préparation d'une composition polyisocyanate de très basse viscosité

Dans cet essai, le bis néodécanoate de zinc est utilisé comme catalyseur unique de synthèse du diluant réactif allophanate (b) et des composés de type biuret (a).

On procède comme pour l'exemple 1 à la différence des quantités de réactifs utilisés qui sont présentés dans le tableau 31. Le bloqueur utilisé est le dibutyl phosphate.

**Tableau 31**

| Réactif | HDI | Eau | 1-butanol | bis-néodécanoate de zinc | Acide Di butyl phosphate |
|---|---|---|---|---|---|
| Quantité en g | 600 | 5 | 4,26 | 0,15 | 0,15 |

Les ratios molaires sont présentés dans le tableau 32.

**Tableau 32**

| NCO/OH alcool | NCO / eau | Métal / NCO | Métal / OH alcool |
|---|---|---|---|
| 124 | 25,7 | 52 10⁻⁶ | 0,0064 |

Le titre NCO de la masse réactionnelle avant distillation est de 1,023 mol pour 100 g de milieu réactionnel. 574g de masse réactionnelle sont filtrés pour éliminer 0,2 g d'impuretés solides. Le filtrat est ensuite distillé sous vide à 140 °C pour éliminer le monomère HDI en excès. On obtient 150 g de composition polyisocyanate. Le rendement pondéral récupéré est de 26 %.

Les caractéristiques des produits de l'exemple 10 obtenu après distillation du monomère diisocyanate utilisé en excès sont présentées dans le tableau 33. La fonctionnalité molaire moyenne en NCO est calculée à partir de la composition analysée par chromatographie par perméation de gel GPC - IR.

**Tableau 33**

| Titre NCO % poids | Viscosité mPas 25°C | Fonctionnalité molaire moyenne en NCO | Taux de transformation de HDI en % poids |
|---|---|---|---|
| 23,2 | 686 | 2,8 | 26 |

La répartition des composés est présentée dans le tableau 34.

**Tableau 34**

| Type de composé | Composé de la composition de l'exemple 10 | % poids |
|---|---|---|
| | HDI monomère | 0,28 |
| (d) | Uretidine dione de HDI (HDI Dimère) | 11,2 |
| (b) | Allophanate de HDI et de n butyle | 14,2 |
| (a) | Dimère-biuret, Biuret (n=3, n=5, n> 5) | 69.3 |
| (c) | Dérivés polyisocyanates biuret- allophanate de HDI et de n butyle | 5 |

Dans le cas de l'exemple 10 la fraction molaire de fonctions allophanates des composés (c) est de 0,0056 pour 150 g de composition.

### Exemple 11 : préparation d'une composition polyisocyanate de basse viscosité

Dans cet essai, le bis néodécanoate de zinc est utilisé comme catalyseur unique de synthèse du diluant réactif allophanate (b) et des composés de type biuret (a).

On procède comme pour l'exemple 1 à la différence des quantités de réactifs utilisés qui sont présentés dans le tableau 35

**Tableau 35**

| Réactif | HDI | Eau | 1-butanol | bis-néodécanoate de zinc | Acide dibutyle phosphate |
|---|---|---|---|---|---|
| Quantité en g | 600 | 7,5 | 4,69 | 0,15 | 0,15 |

Les ratios molaires sont présentés dans le tableau 36.

**Tableau 36**

| NCO/OH alcool | NCO / eau | Métal / NCO | Métal / OH alcool |
|---|---|---|---|
| 113 | 17,2 | 52 10⁻⁶ | 0,0058 |

Le titre NCO de la masse réactionnelle avant distillation est de 0,893 mol pour 100 g de milieu réactionnel. 562,5g de masse réactionnelle sont filtrés pour éliminer 0,16 g d'impuretés solides. Le filtrat est ensuite distillé sous vide à 140 °C pour éliminer le monomère HDI en excès. On obtient 247 g de composition polyisocyanate. Le rendement pondéral récupéré est de 44%.

Les caractéristiques des produits de l'exemple 11 obtenu après distillation du monomère diisocyanate utilisé en excès sont présentées dans le tableau 37. La fonctionnalité molaire moyenne en NCO est calculée à partir de la composition analysée par chromatographie par perméation de gel GPC - IR.

**Tableau 37**

| Titre NCO % poids | Viscosité mPas 25°C | Fonctionnalité molaire moyenne en NCO | Taux de transformation du HDI en % poids |
|---|---|---|---|
| 21,6 | 3157 | 3,2 | 47 |

La répartition des composés est présentée dans le tableau 38.

**Tableau 38**

| Type de composé | Composé de la composition de l'exemple 11 | % poids |
|---|---|---|
| | HDI monomère | 0,4 |
| (d) | Uretidine dione de HDI (HDI Dimère) | 4,5 |
| (b) | Allophanate de HDI et de n butyle | 9,4 |
| (a) | Dimère-biuret, Biuret (n=3, n=5, n> 5) | 78.9 |
| (c) | Dérivés polyisocyanates biuret- allophanate de HDI et de n butyle | 6,4 |

Les composés (a) représentent environ 79 % poids de la composition de l'exemple 11. Dans le cas de l'exemple 11 la fraction molaire de fonctions allophanates des composés (c) est de 0,0017 pour 247 g de composition.

### Exemple 12 : Viscosité des composés (b)

Apres avoir synthétisé différents composés (b), les caractéristiques de ces composés ont ensuite été mesurées et sont présentées dans le tableau 39. La viscosité est mesurée au Rhéomat 300 module 114.

**Tableau 39**

| Nature de l'alcool | Teneur en HDI monomère % poids | Titre NCO % poids | Viscosité mPas à 25°C |
|---|---|---|---|
| Butan 1 ol | 0,05 | 18,6 | 98 |
| Propan 2 ol (IPA) | 0,1 | 20 | 124 |
| Butan 2 ol | 0,5 | 19,4 | 124,7 |
| Cyclohexanol | 0,05 | 17,6 | 406 |

### Exemple 13 : Préparation d'une composition polyisocyanate de viscosité moyenne

On procède comme pour l'exemple 2 mais en utilisant les quantités de réactifs indiquées dans le tableau 40.

**Tableau 40**

| Réactif | HDI | Eau | 1-butanol | bNZ |
|---|---|---|---|---|
| Quantité en g | 600 | 14 | 9 | 0,15 |

Les ratios molaires sont présentés dans le tableau 41.

**Tableau 41**

| NCO / OH alcool | NCO / eau | bNZ/ NCO | bNZ / OH alcool | bNZ/ OH totaux |
|---|---|---|---|---|
| 59,5 | 9,2 | 52.10⁻⁶ | 0,0031 | 4,1 10⁻⁴ |

La quantité d'agent de blocage di butyle phosphate correspond à deux fois la quantité molaire de catalyseur.

La quantité de polyisocyanate récupérée après réaction et distillation du monomère diisocyanate est de 249,5 g correspondant à un rendement pondéral récupéré de 55,9 %. La masse réactionnelle engagée en distillation est de 466 g. Le titre NCO du milieu réactionnel avant distillation est de 0,778 mol pour 100 g.

Les caractéristiques des produits obtenus après distillation du monomère diisocyanate utilisé en excès sont présentées dans le tableau 42.

**Tableau 42**

| Titre NCO % poids | Viscosité mPas 25°C | Fonctionnalité molaire moyenne en NCO | Taux de transformation de HDI en % poids |
|---|---|---|---|
| 19,7 | 21077 | >4 | 63,9 |

### Exemple 14 : Préparation d'une composition polyisocyanate de viscosité moyenne

On procède comme pour l'exemple 2 mais en utilisant les quantités de réactifs indiquées dans le tableau 43.

**Tableau 43**

| Réactif | HDI | Eau | 1-butanol | bNZ |
|---|---|---|---|---|
| Quantité en g | 600 | 10,7 | 5,9 | 0,15 |

Les ratios molaires sont présentés dans le tableau 44.

**Tableau 44**

| NCO / OH alcool | NCO / eau | bNZ/ NCO | bNZ / OH alcool | bNZ / OH totaux |
|---|---|---|---|---|
| 89,5 | 12 | 52.10⁻⁶ | 0,0046 | 5,5.10⁻⁴ |

La quantité d'agent de blocage di butyle phosphate correspond à trois fois la quantité molaire de catalyseur.

La quantité de polyisocyanate récupérée après réaction et distillation du monomère diisocyanate est de 249,5 g correspondant à un rendement pondéral récupéré de 55,9 %. La masse réactionnelle engagée en distillation est de 504 g. Le titre NCO du milieu réactionnel avant distillation est de 0,848 mol pour 100 g.

Les caractéristiques des produits obtenus après distillation du monomère diisocyanate utilisé en excès sont présentées dans le tableau. La fonctionnalité molaire moyenne en NCO est calculée à partir de la composition analysée par chromatographie par perméation de gel GPC - IR.

**Tableau 45**

| Titre NCO % poids | Viscosité mPas 25°C | Taux de transformation de HDI en % poids |
|---|---|---|
| 19,7 | 21077 | 53 |

La répartition des composés est présentée dans le tableau 46.

**Tableau 46**

| Type de composé | Composé de la composition de l'exemple 2 | % poids |
|---|---|---|
| | HDI monomère | 0,13 |
| (d) | Uretidine dione de HDI (HDI Dimère) | 3,7 |
| (b) | Allophanate de HDI et de n butyle | 5,1 |
| (a) + (c) | Dimère-biuret, Biuret (n=3, n=5, n> 5) | 91,07 |
| (c) | Dérivés polyisocyanates biuret- allophanate de HDI et de n butyle | 20 |

La fraction molaire de fonctions allophanates des composés (c) est de 0,049 mole pour 249,5 g de composition. On procède au même type de calcul que pour l'exemple 1.

## Revendications

1. Composition dont la fonctionnalité isocyanate moyenne est supérieure à 2,5 et dont le titre NCO est compris entre 10 et 25 % en poids, comprenant
- au moins un composé polyisocyanate à motifs biuret (a) ;
- au moins un composé diluant réactif polaire, protique (b), choisi parmi les composés polyisocyanates à motifs allophanate, dont la viscosité mesurée à 25 °C est inférieure à 500 mPa.s ;
- au moins un composé d'addition (c) d'un composé (a) et d'un composé (b) et
- au moins un composé diluant réactif aprotique (d) choisi parmi un composé de formule (IV), un composé de formule (V), un composé de formule (VI) et un composé de formule (VII) 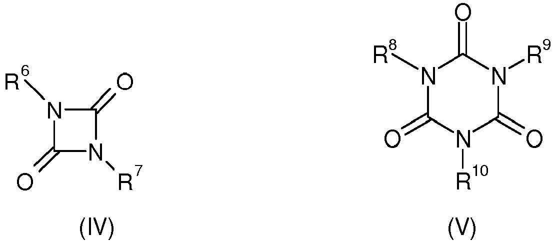 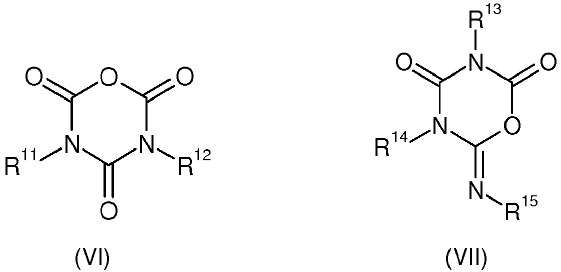
dans lesquelles
a. R⁶ et R⁷, identiques ou différents, représentent indépendamment un groupement C₂-C₂₀ alkyl, linéaire, cyclique ou ramifié, comprenant au moins une fonction isocyanate ; de préférence un groupement C₂-C₁₂ alkyl, linéaire, cyclique ou ramifié, comprenant au moins une fonction isocyanate ; plus préférentiellement un groupement C₂-C₁₂ alkyl, linéaire ou ramifié, comprenant au moins une fonction isocyanate ; en particulier un groupement C₄-C₁₂ alkyl, linéaire ou ramifié, comprenant au moins une fonction isocyanate ;
b. R⁸, R⁹, R¹¹, R¹², R¹³ R¹⁴ et R¹⁵, identiques ou différents, représentent indépendamment un groupement C₂-C₂₀ alkyl, linéaire, cyclique ou ramifié ; de préférence un groupement C₂-C₁₂ alkyl, linéaire, cyclique ou ramifié ; plus préférentiellement un groupement C₂-C₁₂ alkyl, linéaire ou ramifié ; en particulier un groupement C₄-C₁₂ alkyl, linéaire ou ramifié ; un groupement C₂-C₂₀ alkyl, linéaire, cyclique ou ramifié, comprenant au moins une fonction isocyanate ; de préférence un groupement C₂-C₁₂ alkyl, linéaire, cyclique ou ramifié, comprenant au moins une fonction isocyanate ; plus préférentiellement un groupement C₂-C₁₂ alkyl, linéaire ou ramifié, comprenant au moins une fonction isocyanate ; en particulier un groupement C₄-C₁₂ alkyl, linéaire ou ramifié, comprenant au moins une fonction isocyanate.

2. Composition selon la revendication 1 comprenant
∘ de 40 à 50 % en poids de composé (a) ou de 40 à 60 % en poids de composé (a) ou de 40 à 70 % en poids de composé (a) ou de 40 à 80 % en poids de composé (a) ou de 40 à 90 % en poids de composé (a) ;
∘ de 2 à 50 % en poids ou de 5 à 50 % en poids de composé (b) ;
∘ de 0,5 à 20 % en poids ou de 0,5 à 10 % en poids de composé (c) et
∘ de 1 à 20 % en poids de composé (d).

3. Composition selon la revendication 1 ou 2 dont la viscosité mesurée à 25 °C est inférieure à 30 000 mPa.s, de préférence inférieure à 10 000 mPa.s., de préférence inférieure à 5 000 mPa.s, de préférence inférieure à 2 000 mPa.s, de préférence inférieure à 1 500 mPa.s, de préférence inférieure à 1 200 mPa.s, de préférence inférieure à 1 000 mPa.s.

4. Composition selon l'une quelconque des revendications 1 à 3 dont la fonctionnalité isocyanate moyenne est supérieure à 2,75, de préférence supérieure à 2,8, de préférence supérieure à 3.

5. Composition selon l'une des revendications 1 à 4 comprenant indépendamment au moins un composé (a) et au moins un composé (b) préparés à partir d'un composé monomère isocyanate choisi parmi le MPDI, HDI, tetraméthylène-diisocyanate, pentaméthylène-diisocyanate, octaméthylène-diisocyanate, butylène-diisocyanate, octylène-diisocyanate, triméthtylhexane diisocyanate, dodécane-diisocyanate, undécane-diisocyanate, 2,2,4-tris-méthyl-hexaméthylène-diisocyanate, 2,4,4-tris-méthyl-hexaméthylène-diisocyanate, 1,8-diisocyanato-4-isocyanato-méthyl-octane, 1-décane-triisocyanate, IPDI, XDI, MXDI, PXDI, H₁₂MDI, H₆TDI, dérivés de lysine diisocyanate, les BIC et les NBDI.

6. Composition selon l'une des revendications 1 à 5 dans laquelle le composé (b) est un composé de formule (I) 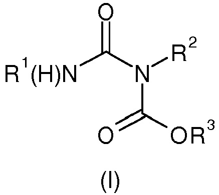 dans laquelle
▪ R¹ et R², identiques ou différents, représentent indépendamment un groupement C₂-C₂₀ alkyl, linéaire, cyclique ou ramifié, comprenant au moins une fonction isocyanate ; de préférence un groupement C₂-C₁₂ alkyl, linéaire, cyclique ou ramifié, comprenant au moins une fonction isocyanate ; plus préférentiellement un groupement C₂-C₁₂ alkyl, linéaire ou ramifié, comprenant au moins une fonction isocyanate ; en particulier un groupement C₄-C₁₂ alkyl, linéaire ou ramifié, comprenant au moins une fonction isocyanate ;
▪ R³ représente indépendamment un groupement C₅-C₁₀ hétérocycloalkyl ; un groupement C₅-C₁₀ aromatique ; un groupement C₅-C₁₀ alkyl-aryl ; un groupement C₁-C₂₀ alkyl, linéaire, cyclique ou ramifié ; de préférence un groupement C₁-C₁₂ alkyl, linéaire, cyclique ou ramifié ; plus préférentiellement un groupement C₁-C₁₂ alkyl, linéaire ou ramifié ; notamment un groupement C₁-C₁₂ alkyl, linéaire ou ramifié ; en particulier un groupement C₃-C₈ alkyl, linéaire ou ramifié.

7. Composition selon la revendication 6 dans laquelle dans laquelle le composé (b) est un composé de formule (I) dans laquelle
▪ R¹ et R², identiques ou différents, représentent indépendamment un groupement C₂-C₈ alkyl, linéaire ou ramifié, comprenant une fonction isocyanate ; de préférence un groupement hexyl comprenant une fonction isocyanate,
▪ R³ représente indépendamment un groupement C₃-C₈ alkyl, linéaire ou ramifié ; de préférence un groupement choisi parmi propyl, butyl, hexyl, octyl, 2-éthyl-hexyl ou un groupement C₅-C₁₀ hétérocycloalkyl comprenant au moins un hétéroatome choisi parmi O, S et N.

8. Procédé de préparation d'une composition selon l'une quelconque des revendications 1 à 7 comprenant les étapes suivantes :
1) préparation en présence d'un catalyseur unique choisi parmi les carboxylates de zinc et les alkylcarboxylates de zinc, d'au moins un composé polyisocyanate à motifs biuret (a) ;
2) préparation en présence d'un catalyseur unique choisi parmi les carboxylates de zinc et les alkylcarboxylates de zinc, d'au moins un composé diluant réactif polaire, protique (b) choisi parmi les composés polyisocyanates à motifs allophanate, dont la viscosité mesurée à 25 °C est inférieure à 500 mPa.s;
3) préparation d'au moins un composé d'addition (c) ;
4) préparation d'au moins un composé diluant réactif polaire, protique (d) ; puis
5) séparation du monomère isocyanate en excès.

9. Procédé selon la revendication 8 dans lequel l'étape 1), 2) ou 4) peut être réalisée en premier ou dans lequel les étapes 1) et 2) ou 2) et 3) ou 3) et 4) ou 1) et 2) et 3) et 4) peuvent être réalisées simultanément.

10. Procédé selon l'une des revendications 8 ou 9 dans lequel le ratio molaire somme du ou des catalyseurs /nombre de fonctions OH est compris entre 1.10⁻² et 1.10⁻⁵.

11. Utilisation d'au moins une composition selon l'une quelconque des revendications 1 à 7 comme durcisseur pour la préparation d'un revêtement ou d'un adhésif ; de préférence pour la préparation d'un revêtement polyuréthane, d'un revêtement polyurée, d'un revêtement poly(urée-uréthane), d'un adhésif polyuréthane, d'un adhésif polyurée ou d'un adhésif poly(urée-uréthane).

## Patentansprüche

1. Zusammensetzung, deren mittlere Isocyanatfunktionalität größer als 2,5 ist und deren NCO-Gehalt zwischen 10 und 25 Gew.-% liegt, umfassend
- mindestens eine Polyisocyanatverbindung mit Biureteinheiten (a);
- mindestens eine polare, protische reaktive Lösungsmittelverbindung (b), ausgewählt unter Polyisocyanatverbindungen mit Allophanateinheiten, deren bei 25 °C gemessene Viskosität weniger als 500 mPa.s beträgt;
- mindestens eine Additionsverbindung (c) aus einer Verbindung (a) und einer Verbindung (b) und
- mindestens eine aprotische reaktive Lösungsmittelverbindung (d), ausgewählt unter einer Verbindung mit der Formel (IV), einer Verbindung mit der Formel (V), einer Verbindung mit der Formel (VI) und einer Verbindung mit der Formel (VII) 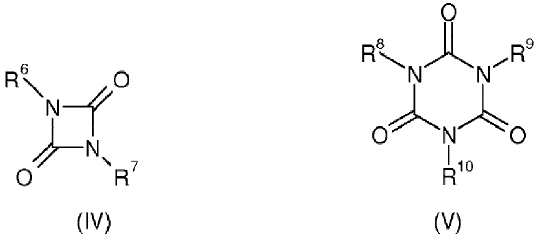 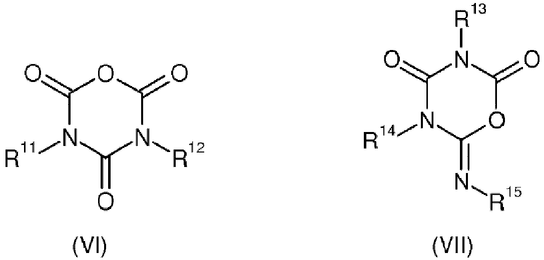
in denen
a. R⁶ und R⁷, gleich oder verschieden, unabhängig voneinander für eine geradkettige, ringförmige oder verzweigte C₂-C₂₀-Alkylgruppe, umfassend mindestens eine Isocyanatfunktion; vorzugsweise eine geradkettige, ringförmige oder verzweigte C-₂-C₁₂-Alkylgruppe, umfassend mindestens eine Isocyanatfunktion; stärker bevorzugt eine geradkettige oder verzweigte C₂-C₁₂-Alkylgruppe, umfassend mindestens eine Isocyanatfunktion; insbesondere eine geradkettige oder verzweigte C₄-C₁₂-Alkylgruppe, umfassend mindestens eine Isocyanatfunktion, stehen;
b. R⁸, R⁹, R¹¹, R¹², R¹³, R¹⁴ und R¹⁵, gleich oder verschieden, unabhängig voneinander für eine geradkettige, ringförmige oder verzweigte C₂-C₂₀-Alkylgruppe; vorzugsweise eine geradkettige, ringförmige oder verzweigte C₂-C₁₂-Alkylgruppe; stärker bevorzugt eine geradkettige oder verzweigte C₂-C₁₂-Alkylgruppe; insbesondere eine geradkettige oder verzweigte C₄-C₁₂-Alkylgruppe; eine geradkettige, ringförmige oder verzweigte C₂-C₂₀-Alkylgruppe, umfassend mindestens eine Isocyanatfunktion; vorzugsweise eine geradkettige, ringförmige oder verzweigte C₂-C₁₂-Alkylgruppe, umfassend mindestens eine Isocyanatfunktion; stärker bevorzugt eine geradkettige oder verzweigte C₂-C₁₂-Alkylgruppe, umfassend mindestens eine Isocyanatfunktion; insbesondere eine geradkettige oder verzweigte C₄-C₁₂-Alkylgruppe, umfassend mindestens eine Isocyanatfunktion, stehen.

2. Zusammensetzung nach Anspruch 1, umfassend
∘ 40 bis 50 Gew.-% der Verbindung (a) oder 40 bis 60 Gew.-% der Verbindung (a) oder 40 bis 70 Gew.-% der Verbindung (a) oder 40 bis 80 Gew.-% der Verbindung (a) oder 40 bis 90 Gew.-% der Verbindung (a);
o 2 bis 50 Gew.-% oder 5 bis 50 Gew.-% der Verbindung (b);
o 0,5 bis 20 Gew.-% oder 0,5 bis 10 Gew.-% der Verbindung (c); und
o 1 bis 20 Gew.-% der Verbindung (d).

3. Zusammensetzung nach Anspruch 1 oder 2, deren bei 25 °C gemessene Viskosität weniger als 30 000 mPa.s, vorzugsweise weniger als 10 000 mPa.s, vorzugsweise weniger als 5 000 mPa.s, vorzugsweise weniger als 2 000 mPa.s, vorzugsweise weniger als 1 500 mPa.s, vorzugsweise weniger als 1 200 mPa.s, vorzugsweise weniger als 1 000 mPa.s beträgt.

4. Zusammensetzung nach einem der Ansprüche 1 bis 3, deren mittlere Isocyanatfunktionalität mehr als 2,75, vorzugsweise mehr als 2,8, vorzugsweise mehr als 3 beträgt.

5. Zusammensetzung nach einem der Ansprüche 1 bis 4, umfassend unabhängig voneinander mindestens eine Verbindung (a) und mindestens eine Verbindung (b), hergestellt aus einer monomeren Isocyanatverbindung, ausgewählt unter MPDI, HDI, Tetramethylendiisocyanat, Pentamethylendiisocyanat, Octamethylendiisocyanat, Butylendiisocyanat, Octylendiisocyanat, Trimethylhexandiisocyanat, Dodecandiisocyanat, Undecandiisocyanat, 2,2,4-Trimethylhexamethylendiisocyanat, 2,4,4-Trimethylhexamethylendiisocyanat, 1,8-Diisocyanat-4-isocyanatmethyloctan, 1-Decantriisocyanat, IPDI, XDI, MXDI, PXDI, H₁₂MDI, H₆TDI, Lysindiisocyanat-Derivaten, den BIC und den NBDI.

6. Zusammensetzung nach einem der Ansprüche 1 bis 5, wobei die Verbindung (b) eine Verbindung mit der Formel (I) ist 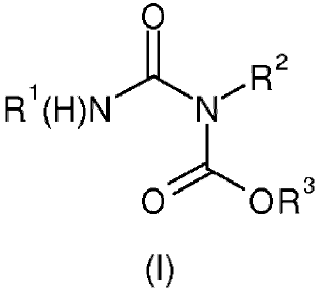 in der
▪ R¹ und R², gleich oder verschieden, unabhängig voneinander für eine geradkettige, ringförmige oder verzweigte C₂-C₂₀-Alkylgruppe, umfassend mindestens eine Isocyanatfunktion; vorzugsweise eine geradkettige, ringförmige oder verzweigte C₂-C₁₂-Alkylgruppe, umfassend mindestens eine Isocyanatfunktion; stärker bevorzugt eine geradkettige oder verzweigte C₂-C₁₂-Alkylgruppe, umfassend mindestens eine Isocyanatfunktion; insbesondere eine geradkettige oder verzweigte C₄-C₁₂-Alkylgruppe, umfassend mindestens eine Isocyanatfunktion, stehen;
▪ R³ unabhängig für eine C₅-C₁₀-Heterocycloalkylgruppe; eine aromatische C₅-C₁₀-Gruppe; eine C₅-C₁₀-Alkylarylgruppe; eine geradkettige, ringförmige oder verzweigte C₁-C₂₀-Alkylgruppe; vorzugsweise eine geradkettige, ringförmige oder verzweigte C₁-C₁₂-Alkylgruppe; stärker bevorzugt eine geradkettige oder verzweigte C₁-C₁₂-Alkylgruppe; insbesondere eine geradkettige oder verzweigte C₁-C₁₂-Alkylgruppe; insbesondere eine geradkettige oder verzweigte C₃-C₈-Alkylgruppe steht.

7. Zusammensetzung nach Anspruch 6, wobei die Verbindung (b) eine Verbindung mit der Formel (I) ist, in der
▪ R¹ und R², gleich oder verschieden, unabhängig voneinander für eine geradkettige oder verzweigte C₂-C₈-Alkylgruppe, umfassend eine Isocyanatfunktion; vorzugsweise eine Hexylgruppe, umfassend eine Isocyanatfunktion, stehen,
▪ R³ unabhängig für eine geradkettige oder verzweigte C-₃-C₈-Alkylgruppe; vorzugsweise eine Gruppe ausgewählt unter Propyl, Butyl, Hexyl, Octyl, 2-Ethylhexyl oder einer C-₅-C₁₀-Heterocycloalkylgruppe, umfassend mindestens ein Heteroatom, ausgewählt unter O, S und N, steht.

8. Verfahren zur Herstellung einer Zusammensetzung nach einem der Ansprüche 1 bis 7, umfassend die folgenden Schritte:
1) Herstellung von mindestens einer Polyisocyanatverbindung mit Biureteinheiten (a) in Gegenwart eines einzelnen Katalysators, ausgewählt unter Zinkcarboxylaten und Zinkalkylcarboxylaten;
2) Herstellung mindestens einer polaren, protischen reaktiven Lösungsmittelverbindung (b), ausgewählt unter Polyisocyanatverbindungen mit Allophanateinheiten, deren bei 25 °C gemessene Viskosität weniger als 500 mPa.s beträgt, in Gegenwart eines einzelnen Katalysators, ausgewählt unter Zinkcarboxylaten und Zinkalkylcarboxylaten;
3) Herstellung mindestens einer Additionsverbindung (c);
4) Herstellung mindestens einer polaren, protischen reaktiven Lösungsmittelverbindung (d); dann
5) Abtrennung des überschüssigen Isocyanatmonomers.

9. Verfahren nach Anspruch 8, bei dem der Schritt 1), 2) oder 4) zuerst ausgeführt werden kann oder bei dem die Schritte 1) und 2) oder 2) und 3) oder 3) und 4) oder 1) und 2) und 3) und 4) gleichzeitig durchgeführt werden können.

10. Verfahren nach einem der Ansprüche 8 oder 9, bei dem das Molverhältnis Summe des Katalysators oder der Katalysatoren / Anzahl der OH-Funktionen zwischen 1,10⁻² und 1,10⁻⁵ liegt.

11. Verwendung mindestens einer Zusammensetzung nach einem der Ansprüche 1 bis 7 als Härter zur Herstellung einer Beschichtung oder eines Klebstoffs; vorzugsweise zur Herstellung einer Polyurethan-Beschichtung, einer Polyharnstoff-Beschichtung, einer Poly(urethan-harnstoff)-Beschichtung, eines Polyurethan-Klebstoffs, eines Polyharnstoff-Klebstoffs oder eines Poly(urethan-harnstoff)-Klebstoffs.

## Claims

1. A composition, the average isocyanate functionality of which is greater than 2.5 and for which the NCO titre is between 10 and 25% by weight, comprising
- at least one polyisocyanate compound with biuret units (a);
- at least one polar, protic reactive diluent compound (b), chosen from polyisocyanate compositions with allophanate units, the viscosity of which measured at 25°C is less than 500 mPa.s;
- at least one addition compound (c), a compound (a) and a compound (b) and
- at least one aprotic reactive diluent compound (d) chosen from a compound of formula (IV), a compound of formula (V), a compound of formula (VI) and a compound of formula (VII) 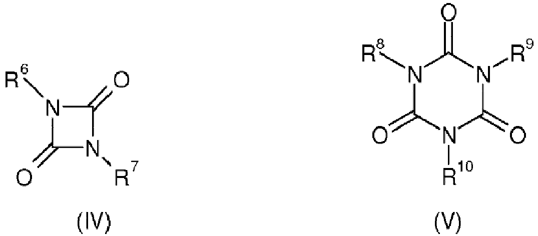 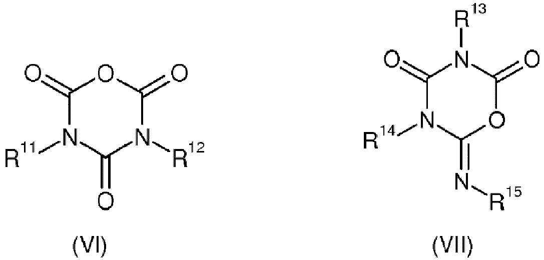
wherein
a. R⁶ and R⁷, identical or different, independently represent a linear, cyclic or branched C₂-C₂₀ alkyl group, comprising at least one isocyanate function; preferably a linear, cyclic or branched C₂-C₁₂ alkyl group, comprising at least one isocyanate function; more preferably a linear or branched C₂-C₁₂ alkyl group, comprising at least one isocyanate function; in particular a linear or branched C₄-C₁₂ alkyl group, comprising at least one isocyanate function;
b. R⁸,R⁹,R¹¹,R¹²,R¹³ R¹⁴ and R¹⁵, identical or different, independently represent a linear, cyclic or branched C₂-C₂₀ alkyl group; preferably a linear, cyclic or branched C₂-C₁₂ alkyl group; more preferably a linear or branched C₂-C₁₂ alkyl group; in particular a linear or branched C₄-C₁₂ alkyl group; a linear, cyclic or branched C₂-C₂₀ alkyl group, comprising at least one isocyanate function; preferably a linear, cyclic or branched C₂-C₁₂ alkyl group, comprising at least one isocyanate function; more preferably a linear or branched C₂-C₁₂ alkyl group, comprising at least one isocyanate function; in particular a linear or branched C₄-C₁₂ alkyl group, comprising at least one isocyanate function.

2. The composition according to claim 1 comprising
∘ 40 to 50% by weight of compound (a), or 40 to 60% by weight of compound (a), or 40 to 70% by weight of compound (a), or 40 to 80% by weight of compound (a), or 40 to 90% by weight of compound (a);
∘ 2 to 50% by weight, or 5 to 50% by weight of compound (b);
∘ 0.5 to 20% by weight, or 0.5 to 10% by weight of compound (c) and
∘ 1 to 20% by weight of compound (d).

3. The composition according to claim 1 or 2, the viscosity of which measured at 25°C is less than 30,000 mPa.s, preferably less than 10,000 mPa.s, preferably less than 5000 mPa.s, preferably less than 2000 mPa.s, preferably less than 1500 mPa.s, preferably less than 1200 mPa.s, preferably less than 1000 mPa.s.

4. The composition according to any one of claims 1 to 3, the average isocyanate functionality of which is greater than 2.75, preferably greater than 2.8, preferably greater than 3.

5. The composition according to one of claims 1 to 4 independently comprising at least one compound (a) and at least one compound (b) prepared from an isocyanate monomer compound chosen from MPDI, HDI, tetramethylene-diisocyanate, pentamethylene-diisocyanate, octamethylene-diisocyanate, butylene-diisocyanate, octylene-diisocyanate, trimethylhexane diisocyanate, dodecane-diisocyanate, undecane-diisocyanate, 2,2,4-tris-methyl-hexamethylene-diisocyanate, 2,4,4-tris-methyl-hexamethylene-diisocyanate, 1,8-diisocyanato-4-isocyanato-methyl-octane, 1-decane-triisocyanate, IPDI, XDI, MXDI, PXDI, H₁₂MDI, H₆TDI, lysine diisocyanate derivatives, BIC and NBDI.

6. The composition according to any one of claims 1 to 5, wherein the compound (b) is a compound of formula (I) 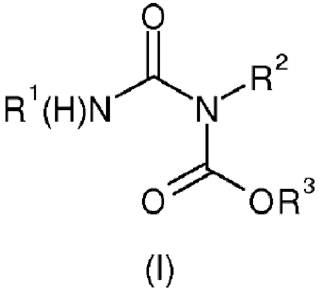 wherein
▪ R¹ and R², identical or different, independently represent a linear, cyclic or branched C₂-C₂₀ alkyl group, comprising at least one isocyanate function; preferably a linear, cyclic or branched C₂-C₁₂ alkyl group, comprising at least one isocyanate function; more preferably a linear or branched C₂-C₁₂ alkyl group, comprising at least one isocyanate function; in particular a linear or branched C₄-C₁₂ alkyl group, comprising at least one isocyanate function;
▪ R³ independently represents a C₅-C₁₀ heterocycloalkyl group; a C₅-C₁₀ aromatic group; a C₅-C₁₀ alkyl-aryl group; a linear, cyclic or branched C₁-C₂₀ alkyl group; preferably a linear, cyclic or branched C₁-C₁₂ alkyl group; more preferably a linear or branched C₁-C₁₂ alkyl group; notably a linear or branched C₁-C₁₂ alkyl group; in particular a linear or branched C₃-C₈ alkyl group.

7. The composition according to claim 6 wherein compound (b) is a compound of formula (I) wherein
▪ R¹ and R², identical or different, independently represent a linear or branched C₂-C₈ alkyl group, comprising an isocyanate function; preferably a hexyl group comprising an isocyanate function,
▪ R³ independently represents a linear or branched C₃-C₈ alkyl group; preferably a group chosen from propyl, butyl, hexyl, octyl, 2-ethyl-hexyl or a C₅-C₁₀ heterocycloalkyl group comprising at least one heteroatom chosen from O, S and N.

8. A method for preparing the composition according to any one of claims 1 to 7, comprising the following steps:
1) preparation in the presence of a single catalyst, chosen from zinc carboxylates and zinc alkylcarboxylates, of at least one polyisocyanate compound with biuret units (a);
2) preparation in the presence of a single catalyst, chosen from zinc carboxylates and zinc alkylcarboxylates, of at least one polar, protic reactive diluent compound (b) chosen from polyisocyanate compounds with allophanate units, the viscosity of which measured at 25°C is less than 500 mPa.s;
3) preparation of at least one addition compound (c);
4) preparation of at least one polar, protic reactive diluent compound (d); then
5) separation of the excess isocyanate monomer.

9. The method according to claim 8, wherein step 1), 2) or 4) can be carried out first or wherein steps 1) and 2), or 2) and 3), or 3) and 4), or 1) and 2) and 3) and 4) can be carried out simultaneously.

10. The method according to one of claims 8 or 9, wherein the sum molar ratio of the catalyst or catalysts/number of OH functions is between 1.10⁻² and 1.10⁻⁵.

11. A use of at least one composition according to any one of claims 1 to 7 as a hardener for the preparation of a coating or an adhesive; preferably for the preparation of a polyurethane coating, a polyurea coating, a poly(urea-urethane) coating, a polyurethane adhesive, a polyurea adhesive or a poly(urea-urethane) adhesive.
